(19) Europäisches Patentamt
European Patent Office
Office européen des brevets

(11) EP 2 579 022 A1

(12) EUROPEAN PATENT APPLICATION
published in accordance with Art. 153(4) EPC

(43) Date of publication:
10.04.2013 Bulletin 2013/15

(21) Application number: 11786569.1

(22) Date of filing: 20.05.2011

(51) Int Cl.:
*G01N 21/27* (2006.01) *B82Y 15/00* (2011.01)
*G01N 33/543* (2006.01)

(86) International application number:
PCT/JP2011/061634

(87) International publication number:
WO 2011/148870 (01.12.2011 Gazette 2011/48)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR

(30) Priority: 28.05.2010 JP 2010123225

(71) Applicants:
- **Tokyo Institute of Technology**
  **Tokyo 152-8550 (JP)**
- **Nippon Steel & Sumikin Chemical Co., Ltd.**
  **Chiyoda-ku**
  **Tokyo 101-0021 (JP)**

(72) Inventors:
- **KAJIKAWA Kotaro**
  **Yokohama-shi**
  **Kanagawa 226-8503 (JP)**
- **ENOMOTO Yasushi**
  **Kisarazu-shi**
  **Chiba 292-0835 (JP)**
- **MATSUMURA Yasufumi**
  **Kisarazu-shi**
  **Chiba 292-0835 (JP)**
- **SHINTA Ryuzo**
  **Kisarazu-shi**
  **Chiba 292-0835 (JP)**

(74) Representative: **Becker Kurig Straus**
**Bavariastrasse 7**
**80336 München (DE)**

# (54) METAL MICROPARTICLE COMPOSITE AND METHOD FOR PRODUCING THE SAME

(57) A nano-composite 10 is described, including a matrix resin 1, metal fine-particles 3 immobilized in the matrix resin 1, a binding species 7 immobilized on a part or all of the metal fine-particles 3, and metal fine-particles 9 indirectly immobilized on the metal fine-particles 3 via the binding species 7. Each of at least a part of the metal fine-particles 3 has a portion embedded in the matrix resin 1, and a portion (exposed portion 3a) exposed outside of the matrix resin 1, while the binding species 7 is immobilized on the exposed portions.

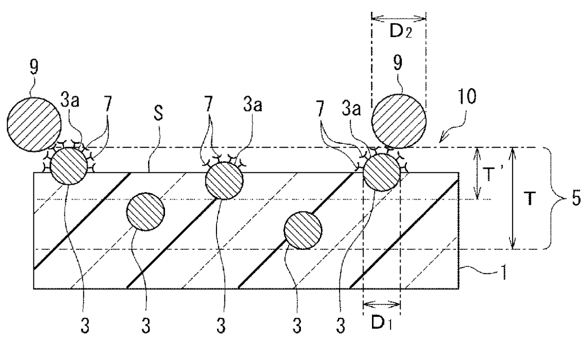


FIG. 1



Printed by Jouve, 75001 PARIS (FR)

## Description

## BACKGROUND OF THE INVENTION

Field of Invention

This invention relates to a metal fine-particle composite that can be utilized in, for example, various devices utilizing local surface plasmon resonance, and to a method for fabricating the same.

Description of Related Art

Local surface plasmon resonance (LSPR) is a phenomenon that the electrons in metal fine-particles or metal fine-structures having a size of several to 100 nanometers interact and resonate with light of a specific wavelength. From ancient periods, LSPR has been utilized in stained glass that shows vivid colors by mixing metal fine-particles into glass. Recently, LSPR was studied for applications such as development of high-output light-emitting laser that utilizes a light-intensity enhancing effect, or bio-sensors that utilize the property that the resonance state is changed by molecular binding, etc.

In order to apply the LSPR of such metal fine-particles to sensors and so on, it is necessary to stably immobilize the metal fine-particles in a matrix of a synthetic resin or the like. However, when the metal fine-particles have a nanometer size, the aggregation/dispersion property thereof changes. For example, dispersion stabilization due to electrostatic repulsion is difficult to achieve so that aggregation occurs easily. Hence, for plasmonic devices utilizing LSPR, how to disperse the metal fine-particles in the matrix in a uniform state is an important issue.

Conventionally, a sensor utilizing plasmon resonance applies the highly sensitive response of the metal plasmon to the refractive index change of the interfacial material, wherein a ligand molecule having a specific interaction with a to-be-detected molecule (analyte) is immobilized by chemical or physical means on the surface of a metal film or metal fine-particles of gold or silver, etc., and the concentration of the analyte is measured. For a SPR sensor utilizing surface plasmon resonance, the technique of applying a metal film formed through sputtering or vacuum evaporation as disclosed in Patent Document 1 has been known.

However, because the technique of Patent Document 1 uses a metal film, in a sensor utilizing surface plasmon resonance, an optical equipment such as a prism or a goniometer, etc., is required as an assist for high-precision sensing. Hence, there is a drawback that miniaturizing the measurement apparatus is difficult or the measurement apparatus is not suitable for simple sensing.

### Prior-Art Documents

### Patent Documents

Patent Document 1: Japanese Patent Publication No. 2006-234472

### Non-Patent Documents

Non-Patent Document 1: Grabar, K. C. et al., Anal. Chem. 1995, 67, 735-743
Non-Patent Document 2: Freeman, R. G. et al, Science 1995, 267, 1629-1632

## SUMMARY OF THE INVENTION

Problems to be Solved by the Invention

When a metal fine-particle composite with metal fine-particles dispersed in a matrix is utilized in applications such as LSPR-based sensors, it is important to have a large absorption spectral intensity at least. Moreover, in general, when the absorption spectrum is sharper, a high-sensitivity detection is more possible.

This invention is devised in view of the above problems, and has an object of providing a metal fine-particle composite having a high-intensity and sharp absorption spectrum of the LSPR.

Means for Solving the Problems

After diligent studying of the above facts, the Inventors discovered that a metal fine-particle composite having first metal fine-particles immobilized in a matrix resin and second metal fine-particles indirectly immobilized on the first

metal fine-particles can meet the above requirements. This invention was accomplished based on the discovery.

Specifically, the metal fine-particle composite of this invention includes a matrix resin and metal fine-particles immobilized to the matrix resin, and is characterized in that

a) the metal fine-particles include a plurality of first metal fine-particles immobilized in the matrix resin, and second metal fine-particles indirectly immobilized on the first metal fine-particles,
b) the first metal fine-particles are present independently without contacting with each other, and
c) each of at least a part of the first metal fine-particles has a portion embedded in the matrix resin and a portion exposed outside of the matrix resin, while the second metal fine-particles are immobilized via a binding species immobilized on the exposed portions.

In the metal fine-particle composite of this invention, the first metal fine-particles may have a particle diameter in the range of 1 nm to 50 nm and a mean particle diameter greater than or equal to 3 nm, and the second metal fine-particles may have a mean particle diameter in the range of 40 nm to 200 nm.

Moreover, in the metal fine-particle composite of this invention, the first metal fine-particles may be present with a distance greater than or equal to the particle diameter of the larger one of two neighboring first metal fine-particles.

Moreover, in the metal fine-particle composite of this invention, another binding species having a functional group interacting with a specific substance may be immobilized on the surfaces of the second metal fine-particles.

Moreover, in the metal fine-particle composite of this invention, the second metal fine-particles may be formed from a metal colloidal.

The method for producing a metal fine-particle composite of this invention includes:

A) a step of contacting the first metal fine-particles, each of which has a portion embedded in the matrix resin and a portion exposed outside of the matrix resin, with a treating liquid containing the binding species at a temperature of 20°C or lower to selectively bind the binding species to the exposed portions; and
B) a step of immobilizing the second metal fine-particles via the above binding species having been immobilized.

The method for producing a metal fine-particle composite of this invention may further include, before the step A,

C) a step of forming a resin film containing a metal ion or a metal salt;
D) a step of thermally reducing the metal ion or the metal salt in the resin film to separate a plurality of first metal fine-particles in the matrix resin and
E) a step of etching the surface of the matrix resin to partially expose the surface of each of at least a part of the first metal fine-particles.
Moreover, the method for producing a metal fine-particle composite of this invention may further include, after the step B,
F) a step of immobilizing, on the surfaces of the second metal fine-particles, another binding species having a functional group interacting with a specific substance.

Moreover, in the method for producing a metal fine-particle composite of this invention, the step B may use a metal colloidal solution containing the second metal fine-particles in a form of metal colloidal.

### Effects of the Invention

Because the metal fine-particle composite of this invention includes first metal fine-particles immobilized in a matrix resin and second metal fine-particles indirectly immobilized on the first metal fine-particles via a binding species, the absorption spectrum of LSPR has a sufficiently large intensity and is sharp, so a high-sensitivity detection is possible by utilizing the composite in applications of various sensors and so on. Moreover, because the first metal fine-particles are immobilized in the matrix resin, they do not peel off from the matrix resin, and are also possible to be suitably applied to arbitrary shapes including curved-surface shapes.
Moreover, when the metal fine-particle composite of this invention further has another binding species immobilized on the second metal fine-particles, it can be applied to certain uses such as the sensors based on the interaction of the another binding species and a specific substance.

## BRIEF DESCRIPTION OF THE DRAWINGS

FIG. 1 schematically illustrates a cross-sectional structure of the nano-composite in the thickness direction of the same.
FIG. 2 illustrates the structure of the metal fine-particles.
FIG. 3 illustrates the state of the second binding species (ligand) being bonded to the nano-composite.
FIG. 4 illustrates the state of the analyte being specifically bonded to the ligand.
FIG. 5 shows an image of the nano-composite film 1b obtained through a surface observation in Example 1.
FIG. 6 shows an image of the nano-composite film 1d obtained through a surface observation in Example 1.
FIG. 7 shows a surface-observation image of the nano-composite film obtained in Reference Example 1.
FIG. 8 shows a surface-observation image of the nano-composite film obtained in Reference Example 2.

## DESCRIPTION OF EMBODIMENTS

The embodiments of this invention will be described in details as follows in reference of appropriate drawings.

<Metal Fine-particle Composite>

FIG. 1 schematically illustrates a cross-sectional structure in the thickness direction of a nano-composite in which metal fine-particles are dispersed (simply referred to as "nano-composite", hereafter) 10 as the metal fine-particle composite according to this embodiment. The nano-composite 10 includes a matrix resin 1, metal fine-particles 3 (first metal fine-particles) immobilized in the matrix resin 1, a binding species 7 immobilized on a part or all of the metal fine-particles 3, and metal fine-particles 9 (second metal fine-particles) indirectly immobilized on the metal fine-particles 3 via the binding species 7. FIG. 2 illustrates, in a magnified view, the metal fine-particles 3 (in the state that the binding species 7 is not bonded). Moreover, in FIG. 2, among two neighboring metal fine-particles 3, the particle diameter of the larger metal fine-particle 3 is designated as $D_{1L}$ and that of the smaller metal fine-particle 3 as $D_{1S}$. However, when the particle diameters are not to be distinguished, the particle diameter is simply designated as $D_1$.

The nano-composite 10 may further have a substrate not shown in the figures. For example, glass, ceramics, a silicon wafer, semiconductor, paper, metal, metal alloy, metal oxide, a synthetic resin, or an organic/inorganic composite material, etc., can be used as the substrate. The shape of the substrate may suitably be a plate shape, a sheet shape, a film shape, a mesh shape, a geometrical pattern shape, a concave-convex shape, a fibrous shape, a bellow shape, a multi-layer shape, or a spherical shape, etc. Moreover, the substrates with surfaces having been subjected to, for example, a silane coupling agent treatment, a chemical etching treatment, a plasma treatment, an alkali treatment, an acid treatment, an ozone treatment, a UV-treatment, an electrical polishing treatment, or a polishing treatment using an abrasive, etc., can also be utilized.

<Matrix Resin>

The matrix resin 1 may be entirely formed in a film shape, or may alternatively be formed as a part of a resin film.

When the matrix resin 1 is formed as a part of a resin film, the thickness of the resin film is preferably in the range of 3 $\mu$m to 100 $\mu$m, more preferably in the range of 10 $\mu$m to 50 $\mu$m.

The resin constituting the matrix resin 1 preferably has a light transparency allowing generation of LSPR of the metal fine-particles 3, and, in particular, preferably includes a material transparent to light with a wavelength greater than or equal to 380 nm. Such matrix resin 1 allows the LSPR to be measured in a light transmission system. On the other hand, a resin almost without a light transparency can also be suitably used as the matrix resin 1, which allows the LSPR to be measured in a light reflection system. Such conformations are not limited to be utilized in light transmission systems and light reflection systems, and may be utilized as sensitivity sensors that sense the change of the circumstance of the matrix resin 1.

The resin materials that may be used as the matrix resin 1 are not particularly limited. Examples thereof include polyimide resins, polyamic acid resins, cardo resins (fluorene resins), polysiloxane resins such as PDMS (polydimethylsiloxane), polyethylene terephthalate resins, polyphenylene ether resins, epoxy resins, fluorine resins, vinyl resins and phenol resins, etc., ion-exchange resins, and so on. Among the resins, those having a functional group that interacts with a metal ion to form a complex with the metal ion and adsorbs the metal ion is preferred, as being able to adsorb the metal ion in a state of uniform dispersion. Examples of such functional groups include carboxyl group, sulfonic acid group, quarternary ammonium groups, primary and secondary amino groups, and phenolic hydroxyl groups, etc. In view of this, for example, polyamic acid resins and ion-exchange resins, etc. are preferred. Moreover, in view of being suitable for the thermal treatment in the separation process of the metal fine-particles 3, the resin preferably has a thermal resistance at a temperature of at least 140°C. In view of this, using the polyimide resin as the material of the matrix resin 1 is particularly preferred because the polyamic acid resin as its precursor has carboxyl groups capable of forming complexes with metal ions so that it can adsorbs metal ions in the precursor stage, and also because it has thermal

resistance to the thermal treatment. Details of the polyimide resin and the polyamic acid resin are described later. Moreover, the above resin material may consist of a single resin, or may alternatively be used as a mixture of a plurality of resins.

<First Metal Fine-particles>

The material of the metal fine-particles 3 as the first metal fine-particles is not particularly limited. For example, a metal species such as gold (Au), silver (Ag), copper (Cu), cobalt (Co), nickel (Ni), palladium (Pd), platinum (Pt), tin (Sn), rhodium (Rh) or iridium (Ir), etc., can be used. Moreover, the alloys of these metal species, such as a Pt-Co alloy, can also be used. Among these materials, silver (Au) or silver (Ag) is particularly preferred.

The metal fine-particles 3 may have various shapes, such as a spherical shape, an ellipsoid shape, a cubic shape, a truncated tetrahedral shape, a bipyramid shape, a regular octahedral shape, a regular decahedral shape, and a regular icosahedral shape, etc. However, in order to inhibit aggregation of the metal fine-particles 9 being the second metal fine-particles and indirectly immobilize the metal fine-particles 9 in a nearly uniform state, the spherical shape is more preferred. Herein, the shape of the metal fine-particles 3 can be identified using a transmission electron microscope (TEM). The spherical metal fine-particles 3 are defined as metal fine-particles having a spherical shape or a nearly spherical shape, wherein the ratio of the average long diameter to the average short diameter is 1 or close to 1 (greater than or equal to 0.8 is preferred). Moreover, regarding the relationship between the long diameter and the short diameter of any individual metal fine-particle 3, it is preferred that the long diameter is less than 1.35 times the short diameter, and is more preferred that the long diameter is equal to or less than 1.25 times the short diameter. Moreover, when the metal fine-particles 3 do not have a spherical shape but have, for example, a regular octahedral shape, the largest one among the edge lengths of a metal fine-particle 3 is taken as the long diameter of the same, the smallest one among the edge lengths is taken as the short diameter of the same, and the above long diameter is considered as the particle diameter $D_1$ of the same.

The metal fine-particles 3 are present independently without contacting with each other, and in particular, are preferably present with a distance greater than or equal to the particle diameter of the larger one of two neighboring metal fine-particles. For example, the distance (inter-particle distance) $L_1$ between two neighboring metal fine-particles 3 is preferably greater than or equal to the particle diameter $D_{1L}$ of the larger one of the neighboring metal fine-particles 3 ($L_1 \geq D_{1L}$). In cases where the distance is in the above range, when the metal fine-particles 9, which are the second metal fine-particles, are being immobilized, it is easy to inhibit aggregation of the metal fine-particles 9 with each other. On the other hand, though the inter-particle distance $L_1$ can be made large without causing particular problems, its upper limit is preferably controlled based on the lower limit of the volume fraction of the metal fine-particles 3, because the inter-particle distance $L_1$ of the metal fine-particles 3 that are made into a dispersion state possibly through thermal diffusion is closely correlated to the particle diameter $D_1$ and the volume fraction of the metal fine-particles 3. When the inter-particle distance $L_1$ is large, i.e., when the volume fraction of the metal fine-particles 3 in the nano-composite 10 is less, the number of the metal fine-particles 9 immobilized on the metal fine-particles 3 is decreased, so that the intensity of the absorption spectrum of the LSPR from the metal fine-particles 9 is low. In such cases, for example, it is preferred that the mean particle diameter of the metal fine-particles 9 as described later is greater than or equal to 80 nm.

For at least a part of the metal fine-particles 3, each of them has a portion embedded in the matrix resin 1 and a portion (exposed portion 3a) exposed outside of the matrix resin 1, while a binding species 7 is immobilized on the exposed portions. Because the metal fine-particle 3 has a portion embedded in the matrix resin 1, it can be immobilized firmly in the matrix resin 1 by the anchoring effect. Moreover, because the metal fine-particles 3 have the exposed portions 3a, the binding species can be immobilized thereon. Accordingly, it is preferred that for all of the metal fine-particles 3 immobilized in the matrix resin 1, each has an exposed portion 3a and has a binding species 7 immobilized on the exposed surface 3a. However, it is feasible that there are metal fine-particles 3 entirely embedded in the matrix resin 1 and have no binding species 7 immobilized thereon. The proportion of the metal fine-particles 3 having exposed portions 3a is set, for example, in terms of the ratio of the total area of the exposed portions 3a to the surface area of the matrix resin 1 (including the surface area of the exposed portions 3a), preferably in the range of 0.1 % to 18% and more preferably in the range of 0.2% to 10%. Moreover, the surface area of the exposed portion 3a of a metal fine-particle 3 can be calculated from the two-dimensional image obtained from an observation using, for example, a field-emitting scanning electron microscope (FE-SEM). Moreover, because the ratio of the total area of the exposed portions 3a to the surface area of the matrix resin 1 is closely correlated to the volume fraction of the metal fine-particles 3, the volume fraction of the metal fine-particles 3 is preferably controlled to be in the preferred range described later.

The metal fine-particles 3 are dispersed into a layer having a certain thickness, in the surface direction parallel with the surface S of the matrix resin 1 (the surface of he nano-composite 10), to form a metal fine-particle layer 5. Though the thickness T of the metal fine-particle layer 5 varies with the particle diameter $D_1$ of the metal fine-particles 3, in the applications utilizing LSPR, the thickness T of the metal fine-particle layer 5 is preferably in the range of 20 nm to 25 $\mu$m, and more preferably in the range of 30 nm to 1 $\mu$m. Herein, "the thickness T of the metal fine-particle layer

5" means, in a cross section in the thickness direction of the matrix resin 1, the thickness from the top end of the most upward positioned metal fine-particle 3 (but which should be one having a particle diameter in the range of 1 nm to 50 nm) at the side of exposure from the matrix resin 1 to the bottom end of the most downward (deeply) positioned metal fine-particle 3 (but which should be one having a particle diameter in the range of 1 nm to 50 nm).

Moreover, in the application of detecting the change of the circumstance of the matrix resin 1, the metal fine-particles 3 entirely embedded in the matrix are almost uncorrelated to the change of the circumstance. In view of this, the thickness T of the metal fine-particle layer 5 constituted by the metal fine-particles 3 is more preferably in the range of 10 nm to 80 nm.

Moreover, when the cross section parallel with the surface S of the matrix resin 1 is observed, it is the most preferred mode of the nano-composite 10 that all the metal fine-particles 3 are completely independent from each other. In such a case, the thickness T' of the metal fine-particle layer 5 constituted only by metal fine-particles 3 having exposed portions 3a is preferably in the range of 20 nm to 40 nm, for example. Moreover, the particle diameter $D_1$ of the metal fine-particles 3 is preferably in the range of 10 nm to 30 nm. By setting the values in the ranges, the intensity of the absorption spectrum of the LSPR from the metal fine-particles 3 can be inhibited to be low. Herein, "the thickness T' of the metal fine-particle layer 5" means, in a cross section in the thickness direction of the matrix resin 1, the thickness from the top end of the most upward positioned one (but which should be one having a particle diameter in the range of 10 nm to 30 nm) of the metal fine-particles 3 having exposed portions 3a at the side of exposure of the matrix resin 1 to the bottom end of the most downward (deeply) positioned one (but which should be one having a particle diameter in the range of 10 nm to 30 nm) of the metal fine-particles 3 having exposed portions 3a.

Moreover, by making the particle diameter $D_1$ of the metal fine-particles 3 present in the matrix resin 1 be greater than or equal to 10 nm, with the metal fine-particles 3 dottedly distributed in a single layer, the relatively larger metal fine particles 9 having a higher intensity of absorption spectrum and a mean particle diameter about 100 nm can be immobilized in a large number without aggregation, so that a detection of higher sensitivity is possible. Herein, that the metal fine-particles 3 are dottedly distributed in a single layer means that they are distributed about two-dimensionally inside of the matrix resin 1. In other words, it is preferred that 90% or more of the total metal fine-particles 3 are dispersed, within the depth of 40 nm from the surface S of the matrix resin, in the plane direction parallel to the surface S to form a metal fine-particle layer 5, and only one metal fine-particle 3 having a particle diameter $D_1$ in the range of 10 nm to 30 nm is present in the depth direction in the metal fine-particle layer 5. That is, though not shown in the figures, when the cross section of the nano-composite 10 parallel to the surface S of the matrix resin 1 is observed, in the interior (or the surface S) of the matrix resin 1, a plurality of metal fine-particles 3 having particle diameters $D_1$ in the range of 10 nm to 30 nm are observed to be dottedly distributed and diffuse with an inter-particle distance $L_1$. Meanwhile, when a cross section along the depth direction of the matrix resin 1 is observed, as shown in FIG. 1, a plurality of metal fine-particles 3 having a particle diameter $D_1$ in the range of 10 nm to 30 nm are completely independently and dottedly distributed into a single layer (substantially in a row, though with a certain positional variation) in the ambit of the metal fine-particle layer 5. Moreover, the method of observing the cross section parallel to the surface S of the matrix resin 1 may be, for example, using a scanning electron microscope (SEM) given a sputtering function to sputter and observe the surface layer of the matrix resin 1.

The existence proportion of the metal fine-particles 3 with particle diameter $D_1$ of from 1 nm to less than 10 nm, which may be measured by observing a cross-section of the matrix resin 1 using a transmission electron microscope (TEM), is preferably less than 50%, more preferably less than 10% and even more preferably less than 1%, relative to the total metal fine-particles 3 in the nano-composite 10. Herein, the "existence proportion" is calculated by dividing the sum of the cross-sectional areas of the metal fine-particles 3 with particle diameters $D_1$ of from 1 nm to less than 10 nm by the sum of the cross-sectional areas of all the metal fine-particles 3. Herein, when metal fine-particles 3 with particle diameters $D_1$ less than 10 nm are present, apparent overlapping of metal fine-particles 3 is identified in the thickness direction of the nano-composite 10. In such a case, relative to the sum of the cross-sectional areas of all the metal fine-particles 3 being observed, the ratio of the sum of the cross-sectional areas of the metal fine-particles 3 observed to be entirely independent from each other is preferably 90% or more, more preferably 95% or more, and even more preferably 99% or more. Moreover, because it is easier to control the surface areas of the exposed portions 3a to be uniform when the particle-diameter distribution of the metal fine-particles 3 is narrower, it is preferred to control the particle-diameter distribution of the metal fine-particles 3 to be narrow. In view of this, about the particle-diameter distribution of the metal fine-particles 3, it is preferred that the maximal particle diameter ($D_{1max}$) and the minimal particle diameter ($D_{1min}$) satisfy the relationship of "$(1/3 \times D_{1max}) < (1 \times D_{1min})$". Moreover, since the particle diameter $D_1$ of the metal fine-particles 3 is closely correlated to the thickness (T') of the metal fine-particle layer 5, it is preferred that the thickness T' (nm) of the metal fine-particle layer 5 and the maximal particle diameter $D_{1max}$ satisfy "$(1/2 \times T') \leq (1xD_{1max})$".

Moreover, it is also feasible that the metal fine-particles 3 are dispersed three-dimensionally inside of the matrix resin 1. That is, when a cross section of the nano-composite 10 along the thickness direction of the film-shaped matrix resin 1 is observed, as shown in FIG. 1, a plurality of metal fine-particles 3 are dottedly distributed in the longitudinal direction and in the transverse direction with an inter-particle distance $L_1$ greater than or equal to the above particle

diameter $D_{1L}$. Meanwhile, when the cross section of the nano-composite 10 parallel with the surface of the matrix resin 1 is observed, though not shown in the figures, a plurality of metal fine-particles 3 is dottedly distributed and diffuses inside of the matrix resin 1 with an inter-particle distance $L_1$ greater than or equal to the above particle diameter $D_{1L}$.

Moreover, it is preferred that 90% or more of the metal fine-particles 3 are single particles dottedly distributed in the matrix resin 1 with an inter-particle distance $L_1$ greater than or equal to the above particle diameter $D_{1L}$. Herein, the term "single particles" means that the metal fine-particles 3 are independently present in the matrix resin 1 but do not include aggregations of multiple particles (aggregated particles). That is, the single particles do not include aggregated particles formed by aggregation of multiple metal fine-particles through an inter-molecular force. Moreover, an "aggregated particle" can be clearly identified by, for example, an observation using a transmission electron microscope (TEM), to be one aggregate formed by an assembly of multiple individual metal fine-particles. Moreover, though in terms of its chemical structure, the metal fine-particles 3 in the nano-composite 10 are known to be metal fine-particles formed by aggregation of metal atoms formed by thermal reduction, they are considered to be formed by metal bonding of metal atoms and be different from the aggregated particles formed by aggregation of multiple particles. For example, when a transmission electron microscope (TEM) is used to observe, a single independent metal fine-particle 3 can be identified. By making the above single particles present in a proportion of 90% or more and preferably 95% or more, the metal fine-particles 9 can be indirectly immobilized uniformly, so that the absorption spectrum of LSPR is sharp and stable, and a high detection sensitivity is obtained. This means, in other words, that the proportion of the aggregated particles or the particles dispersed with an inter-particle distance $L_1$ less than the above particle diameter $D_{1L}$ is less than 10%. Moreover, when the proportion of the aggregated particles or the particles dispersed with an inter-particle distance $L_1$ less than the above particle diameter $D_{1L}$ exceeds 10%, controlling the particle diameters $D_1$ is very difficult.

In the nano-composite 10 of this embodiment, the metal fine-particles 3 as the first metal fine-particles preferably further meet the following requirements i) to ii).

i) The metal fine-particles 3 are preferably obtained by reducing the metal ion or a metal salt contained in the matrix resin 1 or its precursor resin. The reduction method may be light reduction or thermal reduction, etc.; but in view of controlling the particle distance of the metal fine-particles 3, the products obtained by thermal reduction are preferred. Specific contents of the reduction method are described later.

ii) The particle diameter ($D_1$) of the metal fine-particles 3 may be in the range of 1 nm to 50 nm, and is preferably in the range of 3 nm to 30 nm. The mean particle diameter $D_{1A}$ is preferably greater than or equal to 3 nm. The mean particle diameter $D_{1A}$ of the metal fine-particles 3 means the area mean diameter obtained by measuring 100 arbitrary metal fine-particles 3. If the particle diameter ($D_1$) of the metal fine-particles 3 exceeds 50 nm, the number of the metal fine-particles 9 immobilized on the metal fin-particles 3 is decreased, and a sufficient LSPR effect of the metal fine-particles 9 is difficult to obtain. Moreover, in order to distribute the metal fin-particles 3 in a single layer to thereby improve the sensitivity, it is more preferred that the particle diameters $D_1$ of 90% to 100% of the total metal fine-particles 3 are in the range of 10 nm to 30 nm.

When the metal fine-particles 3 are not spherical, the larger their apparent diameter is, the more easily the indirectly immobilized metal fine-particles 9 aggregate. Hence, when the metal fine-particles 3 are not spherical, the particle diameter $D_1$ is preferably 30 nm or less, more preferably 20 nm or less and even more preferably 10 nm or less. Moreover, in cases where the metal fine-particles 3 are not spherical, while respective metal fine-particles 3 present in the matrix resin 1 are compared with each other for the shape, it is preferred that 80% or more, and more preferably 90% or more, of the total metal fine-particles 3 have substantially the same shape, especially in a relative manner.

It is also feasible that metal fine-particles 3 having particle diameters $D_1$ less than 1 nm are present in the nano-composite 10. Such a nano-composite 10 hardly affects the LSPR and hence has no particular problems. Moreover, the amount of the metal fine-particles 3 having particle diameters $D_1$ less than 1 nm, for example in a case where the metal fine-particles 3 are silver fine-particles, is preferably 10 parts by weight or less and more preferably 1 part by weight or less, relative to 100 parts by weight of the total metal fine-particles 3 in the nano-composite 10. Herein, the metal fine-particles 3 having particle diameters $D_1$ less than 1 nm can be observed using, for example, a high-resolution transmission electron microscope.

Moreover, to inhibit aggregation of the metal fine-particles 9 as the second metal fine-particles and indirectly immobilize the same in a nearly uniform state, the mean particle diameter $D_{1A}$ of the metal fine-particles 3 is suitably greater than or equal to 3 nm, preferably between 3 nm and 30 nm, and more preferably between 3 nm and 20 nm.

Moreover, the volume fraction of the metal fine-particles 3 in the matrix resin 1 relative to the nano-composite 10 is preferably 0.1% to 23% and more preferably 0.3% to 12%. Herein, the "volume fraction" is a value indicating the percentage of the total volume of the metal fine-particles 3 in a certain volume of the nano-composite 10. If the volume fraction of the metal fine-particles 3 is less than 0.1%, the effect of this invention is difficult to obtain. On the contrary, if the volume fraction exceeds 23%, the distance (inter-particle distance $L_1$) between two neighboring metal fine-particles 3 is less than the particle diameter $D_{1L}$ of the larger one of two neighboring metal fine-particles 3, so that it is difficult to control the immobilization of the metal fine-particles 9, which are the second metal fine-particles, to be uniform.

<Binding Species>

The binding species 7 in the nano-composite 10 of this embodiment can be defined as, for example, a substance that has a functional group X1 capable of bonding with the metal fine-particles 3 and a functional group Y1 interacting with the metal fine-particles 9. The binding species 7 is not limited to a single molecule, and also covers, for example, a composite constituted of two or more components, etc. The binding species 7 is bonded with the metal fine-particles 3 via the functional group X1 and immobilized on the exposed portions 3a of the metal fine-particles 3. In such a case, the bonding between the functional group X1 and the metal fine-particles 3 indicates, for example, chemical bonding, or physical bonding through adsorption or the like, etc.

The functional group X1 of the binding species 7 is a functional group immobilized on the surfaces of the metal fine-particles 3, which may be immobilized by chemically bonding to the surfaces of the metal fine-particles, or be immobilized through adsorption. Examples of such functional group X1 include monovalent groups such as -SH, $-NH_2$, $-NH_3X$ (X is a halogen atom), -COOH, $-Si(OCH_3)_3$, $-Si(OC_2H_5)_3$, $-SiCl_3$ and $-SCOCH_3$, etc., and divalent groups such as $-S_2-$ and $-S_4-$, etc. The preferred groups among them are those containing one or more sulfur atoms, such as the mercapto group, the sulfide group and the disulfide group, etc.

Moreover, the functional group Y1 of the binding species 7 may be, for example, a substituent capable of bonding with a metal or an inorganic compound such as a metal oxide. Examples of such interaction-enabling functional group Y1 include -SH, $-NH_2$, $-NR_3X$ (R is a hydrogen atom or a $C_1$-$C_6$ alkyl group, and X is a halogen atom), -COOR (R is a hydrogen atom or a $C_1$-$C_6$ alkyl group), $-Si(OR)_3$ (R is a $C_1$-$C_6$ alkyl group), $SiX_3$ (X is a halogen atom), -SCOR (R is a $C_1$-$C_6$ alkyl group), -OH, $-CONH_2$, $-N_3$, -CR=CHR' (R and R' are each independently a hydrogen atom or a $C_1$-$C_6$ alkyl group),- C≡CR (R is a hydrogen atom or a $C_1$-$C_6$ alkyl group), $-PO(OH)_2$, -COR (R is a $C_1$-$C_6$ alkyl group), imidazolyl, and hydroquinolyl, etc.

Specific examples of the binding species 7 include $HS-(CH_2)_n-OH$ (n=11 or 16), $HS-(CH_2)_n-COOH$ (n=10, 11 or 15), $HS-(CH_2)_n-NH_2{\cdot}HCl$ (n=10, 11 or 16), $HS-(CH_2)_{11}-N(CH_3)_3{}^+Cl^-$, $HS-(CH_2)_{11}-PO(OH)_2$, $HS-(CH_2)_{10}-CH(OH)-CH_3$, $HS-(CH_2)_{10}-COCH_3$, $HS-(CH_2)_n-N_3$ (n=10, 11, 12, 16 or 17), $HS-(CH_2)_n-CH=CH_2$ (n=9 or 15), $HS-(CH_2)_4-C{\equiv}CH$, $HS-(CH_2)_n-CONH_2$ (n=10 or 15), $HS-(CH_2)_{11}-(OCH_2CH_2)_n-OCH_2-CONH_2$ (n=3 or 6), $HO-(CH_2)_{11}-S-S-(CH_2)_{11}-OH$, and $CH_3-CO-S-(CH_2)_{11}-(OCH_2CH_2)_n-OH$ (n=3 or 6), etc.

Other examples of the binding species 7 include: heterocyclic compounds having an amino group or a mercapto group, such as 2-amino-1,3,5-triazine-4,6-dithiol, 3-amino-1,2,4-triazole-5-thiol, 2-amino-5-trifluoromethyl-1,3,4-thiadiazole, 5-amino-2-mercaptobenzimidazole, 6-amino-2-mercaptobenzothiazole, 4-amino-6-mercaptopyrazolo[3,4-*d*]pyrimidine, 2-amino-4-methoxybenzothiazole, 2-amino-4-phenyl-5-tetradecylthiazole, 2-amino-5-phenyl-1,3,4-thiadiazole, 2-amino-4-phenylthiazole, 4-amino-5-phenyl-4H-1,2,4-triazole-3-thiol, 2-amino-6-(methylsulfonyl)benzothiazole, 2-amino-4-methylthiazole, 2-amino-5-(methylthio)-1,3,4-thiadiazole, 3-amino-5-methylthio-1H-1,2,4-thiazole, 6-amino-1-methyluracil, 3-amino-5-nitrobenzisothiazole, 2-amino-1,3,4-thiadiazole, 5-amino-1,3,4-thiadiazole-2-thiol, 2-aminothiazole, 2-amino-4-thiazoleacetic acid, 2-amino-2-thiazoline, 2-amino-6-thiocyanatobenzothiazole, DL-$\alpha$-amino-2-thiopheneacetic acid, 4-amino-6-hydroxy-2-mercaptopyrimidine, 2-amino-6-purinethiol, 4-amino-5-(4-pyridyl)-4H-1,2,4-triazole-3-thiol, $N^4$-(2-amino-4-pyrimidinyl)sulfanylamide, 3-aminorhodanine, 5-amino-3-methylisothiazole, 2-amino-$\alpha$-(methoxyimino)-4-thiazoleacetic acid, thioguanine, 5-amino-tetrazole, 3-amino-1,2,4-triazine, 3-amino-1,2,4-triazole, 4-amino-4H-1,2,4-triazole, 2-aminopurine, aminopyrazine, 3-amino-2-pyrazinecarboxylic acid, 3-aminopyrazole, 3-aminopyrazole-4-carbonitrile, 3-amino-4-pyrazolecarboxylic acid, 4-aminopyrazolo[3,4-d]pyrimidine, 2-aminopyridine, 3-aminopyridine, 4-aminopyridine, 5-amino-2-pyridinecarbonitrile, 2-amino-3-pyridinecarboxaldehyde, 2-amino-5-(4-pyridinyl)-1,3,4-thiadiazole, 2-aminopyrimidine, 4-aminopyrimidine, and 4-amino-5-pyrimidinecarbonitrile, etc; and silane coupling agents having an amino group or a mercapto group, such as 3-aminopropyltriethoxysilane, 3-aminopropyltrimethoxysilane, N-2-(aminoethyl)-3-aminopropyltrimethoxysilane, N-2-(aminoethyl)-3-aminopropylmethyldimethoxysilane, 3-triethoxysilyl-N-(1,3-dimethylbutylidene)propylamine, N-phenyl-3-aminopropyltrimethoxysilane, 3-mercaptopropyltriethoxysilane, 3-mercaptopropyltrimethoxysilane, N-2-(mercaptoethyl)-3-mercaptopropyltrimethoxysilane, N-2-(mercaptoethyl)-3-mercaptopropylmethyldimethoxysilane, 3-triethoxysilyl-N-(1,3-dimethylbutylidene)propylmercapto, and N-phenyl-3-mercaptopropyltrimethoxysilane, etc. Moreover, the binding species 7 is not particularly limited to the above compounds, and the binding species 7 may include the above compounds alone or in combination of two or more thereof.

Moreover, the molecular skeleton of the binding species 7 between the functional groups X1 and Y1 includes atoms selected from the group consisting of carbon, oxygen and nitrogen atoms, and may have, e.g., a straight or branched chemical structure with a straight moiety of $C_2$-$C_{20}$, preferably $C_2$-$C_{15}$ and more preferably $C_2$-$C_{10}$, or a cyclic chemical structure. The molecular skeleton may be designed using a single molecule species, or alternatively using two or more molecule species. In an example of suitably applied embodiments where, for example, a detection object molecule or the like is to be effectively detected, it is preferred that the thickness of the molecular mono-film (or molecular monolayer) formed by the binding species 7 is within the range of about 1.3 nm to 3 nm. In view of this, a binding species 7 having a $C_{11}$-$C_{20}$ alkane chain as a molecular skeleton is preferred. In such a case, because the long alkane chain

immobilized on the surface of the metal fine-particle 3 via the functional group X1 extends vertically from the surface to form a molecular mono-film (molecular monolayer), the functional group Y1 suffuses the surface of the molecular mono-film (molecular monolayer). Well-known thiol compounds useful as reagents for forming self-assembly mono-films (SAM) can be suitably used as such binding species 7.

<Second Metal Fine-Particles>

The material of the metal fine-particles 9, which are the second metal fine-particles, is not particularly limited, and may be a metal species such as gold (Au), silver (Ag), copper (Cu), cobalt (Co), nickel (Ni), palladium (Pd), platinum (Pt), tin (Sn), rhodium (Rh), or iridium (Ir), etc. Moreover, the alloys of these metal species, such as Pt-Co alloys, can also be used. Among them, gold (Au), silver (Ag), copper (Cu), palladium (Pd), platinum (Pt), tin (Sn), rhodium (Rh) and iridium (Ir) can be suitably uses as LSPR-effecting metal species, while gold (Au) and silver (Ag) are particularly preferred. The metal fine-particles 9 may include the same metal species of the metal fine-particles 3, or may include a metal species different from that of the metal fine-particles 3. The metal fine-particles 9 are preferably formed from a metal colloidal containing the above metal, for example. In cases using a metal colloidal, for example in a case using a metal gold colloidal, the surface of the metal gold colloidal can be covered by a protective group such as citric acid. That is, the surface of the metal gold fine-particles can be covered by citric acid, as described in the above Non-Patent Document 1 or 2. When the functional group Y1 of the binding species 7 is, for example, an amino group, it can substitute the citric acid and directly chemically bonds with the metal gold fine-particle.

The metal fine-particles 9 may have various shapes, such as a spherical shape, an ellipsoid shape, a cubic shape, a truncated tetrahedral shape, a bipyramid shape, a regular octahedral shape, a regular decahedral shape, and a regular icosahedral shape, etc. However, in order to sharpen the absorption spectrum of the LSPR, the spherical shape is more preferred. Herein, the shape of the metal fine-particles 9 can be identified using a transmission electron microscope (TEM). The spherical metal fine-particles 9 are defined as metal fine-particles having a spherical shape or a nearly spherical shape, wherein the ratio of the average long diameter to the average short diameter is 1 or close to 1 (greater than or equal to 0.8 is preferred). Moreover, about the relationship between the long diameter and the short diameter of any individual metal fine-particle 3, it is preferred that the long diameter is less than 1.35 times the short diameter, and is more preferred that the long diameter is equal to or less than 1.25 times the short diameter. Moreover, when the metal fine-particles 9 do not have a spherical shape but have, for example, a regular octahedral shape, the largest one among the edge lengths of a metal fine-particle 9 is taken as the long diameter of the same, the smallest one among the edge lengths is taken as the short diameter of the same, and the above long diameter is considered as the particle diameter $D_2$ of the same.

The particle diameter $D_2$ of the metal fine-particles 9 is preferably in the range of 30 nm to 250 nm and more preferably in the range of 50 nm to 200 nm. If the particle diameter $D_2$ of the metal fine-particles 9 exceeds 250 nm, the amount (or the number) of the adhering metal fine-particles 9 is decreased, and a sufficient LSPR effect is not obtained. If the particle diameter $D_2$ of the metal fine-particles 9 is less than 30 nm, the function of enhancing the LSPR effect is not obtained. Moreover, the mean particle diameter $D_{2A}$ of the metal fine-particles 9 is preferably in the range of 40 nm to 200 nm and more preferably in the range of 80 nm to 150 nm. Herein, the mean particle diameter $D_{2A}$ of the metal fine-particles 9 means the area-averaged diameter obtained by measuring 100 arbitrary metal fine-particles 9. Moreover, it is more preferred that the particle diameters $D_2$ of 90% to 100% of the total metal fine-particles 9 are in the range of 30 nm to 200 nm.

As shown in FIG. 3, for example, a second binding species (binding species 11) different from the binding species 7 as the first binding species can be immobilized on the surfaces of the metal fine-particles 9. In the nano-composite 10 of this embodiment, the binding species 11 can be defined as, for example, a substance including a functional group X2 capable of binding with the metal fine-particles 9 and a functional group Y2 interacting with a specific substance such as a detection-object molecule or the like. The binding species 11 is not limited to a single molecule, and also covers, for example, a composite constituted of two or more components. The binding species 11 is immobilized on the surfaces of the metal fine-particles 9 via the bonding between the functional group X2 and the metal fine-particles 9. Herein, the bonding between the functional group X2 and the metal fine-particles 9 means a chemical bonding, or a physical bonding such as adsorption and so on. Moreover, the interaction between the functional group Y2 and the specific substance means, for example, a chemical bonding, or a physical bonding such as adsorption and so on, and may alternatively mean that the functional group Y2 is partially or entirely altered (modified or removed, etc.), and so on.

The functional group X2 of the binding species 11 is for immobilizing the binding species 11 on the surfaces of the metal fine-particles 9, and may be immobilized on the surfaces of the metal fine-particles 9 through chemical bonding, or alternatively be immobilized through adsorption. Examples of such functional group X2 include the same divalent groups among the above examples of the functional group X1 of the binding species 7, wherein those containing one or more sulfur atoms, such as the mercapto group, the sulfide group and the disulfide group, etc., are preferred.

Moreover, the functional group Y2 of the binding species 11 may be, for example, a substituent capable of

bonding with a metal, an inorganic compound such as a metal oxide, or an organic compound such as DNA or protein, or alternatively, a leaving group that may leave due to, for example, an acid or an alkali, etc. Examples of the functional group Y2 allowing such interaction include, in addition to those in the examples of the functional group Y1 of the binding species 7, $-SO_3^-X$ (X is an alkali metal), N-hydroxysuccinimide group (-NHS), a Biotin group, $-SO_2CH_2CH_2X$ (X is a halogen atom, $-OSO_2CH_3$, $-OSO_2C_6H_4CH_3$, $-OCOCH_3$, $-SO_3^-$, or pyridium), etc.

Specific examples of the binding species 11 include: $HS-(CH_2)_n-OH$ (n=11 or 16), $HS-(CH_2)_n-COOH$ (n=10, 11 or 15), $HS-(CH_2)_n-COO-NHS$ (n=10, 11 or 15), $HS-(CH_2)_n-NH_2{\cdot}HCl$ (n=10, 11 or 16), $HS-(CH_2)_{11}-NHCO-Biotin$, $HS-(CH_2)_{11}-N(CH_3)_3^+Cl^-$, $HS-(CH_2)_n-SO_3^-Na^+$ (n=10, 11, or 16), $HS-(CH_2)_{11}-PO(OH)_2$, $HS-(CH_2)_{10}-CH(OH)-CH_3$, $HS-(CH_2)_{10}-COCH_3$, $HS-(CH_2)_n-N_3$ (n=10, 11, 12, 16 or 17), $HS-(CH_2)_n-CH=CH_2$ (n=9 or 15), $HS-(CH_2)_4-C{\equiv}CH$, $HS-(CH_2)_n-CONH_2$ (n=10 or 15), $HS-(CH_2)_{11}-(OCH_2CH_2)_n-OCH_2-CONH_2$ (n=3 or 6), $HO-(CH_2)_{11}-S-S-(CH_2)_{11}-OH$, and $CH_3-CO-S-(CH_2)_{11}-(OCH_2CH_2)_n-OH$ (n=3 or 6).

Other examples of the binding species 11 include: heterocyclic compounds having an amino group or a mercapto group, such as 2-amino-1,3,5-triazine-4,6-dithiol, 3-amino-1,2,4-triazole-5-thiol, 2-amino-5-trifluoromethyl-1,3,4-thiadiazole, 5-amino-2-mercaptobenzimidazole, 6-amino-2-mercaptobenzothiazole, 4-amino-6-mercaptopyrazolo[3,4-*d*]pyrimidine, 2-amino-4-methoxybenzothiazole, 2-amino-4-phenyl-5-tetradecylthiazole, 2-amino-5-phenyl-1,3,4-thiadiazole, 2-amino-4-phenylthiazole, 4-amino-5-phenyl-4H-1,2,4-triazole-3-thiol, 2-amino-6-(methylsulfonyl)benzothiazole, 2-amino-4-methylthiazole, 2-amino-5-(methylthio)-1,3,4-thiadiazole, 3-amino-5-methylthio-1H-1,2,4-thiazole, 6-amino-1-methyluracil, 3-amino-5-nitrobenzisothiazole, 2-amino-1,3,4-thiadiazole, 5-amino-1,3,4-thiadiazole-2-thiol, 2-aminothiazole, 2-amino-4-thiazoleacetic acid, 2-amino-2-thiazoline, 2-amino-6-thiocyanatobenzothiazole, DL-$\alpha$-amino-2-thiopheneacetic acid, 4-amino-6-hydroxy-2-mercaptopyrimidine, 2-amino-6-purinethiol, 4-amino-5-(4-pyridyl)-4H-1,2,4-triazole-3-thiol, $N^4$-(2-amino-4-pyrimidinyl)sulfanylamide, 3-aminorhodanine, 5-amino-3-methylisothiazole, 2-amino-$\alpha$-(methoxyimino)-4-thiazoleacetic acid, thioguanine, 5-amino-tetrazole, 3-amino-1,2,4-triazine, 3-amino-1,2,4-triazole, 4-amino-4H-1,2,4-triazole, 2-aminopurine, aminopyrazine, 3-amino-2-pyrazinecarboxylic acid, 3-aminopyrazole, 3-aminopyrazole-4-carbonitrile, 3-amino-4-pyrazolecarboxylic acid, 4-aminopyrazolo[3,4-*d*]pyrimidine, 2-aminopyridine, 3-aminopyridine, 4-aminopyridine, 5-amino-2-pyridinecarbonitrile, 2-amino-3-pyridinecarboxaldehyde, 2-amino-5-(4-pyridinyl)-1,3,4-thiadiazole, 2-aminopyrimidine, 4-aminopyrimidine, and 4-amino-5-pyrimidinecarbonitrile, etc; and silane coupling agents having an amino group or a mercapto group, such as 3-aminopropyltriethoxysilane, 3-aminopropyltrimethoxysilane, N-2-(aminoethyl)-3-aminopropyltrimethoxysilane, N-2-(aminoethyl)-3-aminopropylmethyldimethoxysilane, 3-triethoxysilyl-N-(1,3-dimethylbutylidene)propylamine, N-phenyl-3-aminopropyltrimethoxysilane, 3-mercaptopropyltriethoxysilane, 3-mercaptopropyltrimethoxysilane, N-2-(mercaptoethyl)-3-mercaptopropyltrimethoxysilane, N-2-(mercaptoethyl)-3-mercaptopropylmethyldimethoxysilane, 3-triethoxysilyl-N-(1,3-dimethylbutylidene)propylmercapto, and N-phenyl-3-mercaptopropyltrimethoxysilane, etc. Moreover, the binding species 11 is not particularly limited to the above compounds, and the binding species 11 may include the above compounds alone or in combination of two or more thereof.

Moreover, the molecular skeleton of the binding species 11 between the functional groups X2 and Y2 includes atoms selected from the group consisting of carbon, oxygen and nitrogen atoms, and may have, e.g., a straight or branched chemical structure with a straight moiety of $C_2$-$C_{20}$, preferably $C_2$-$C_{15}$ and more preferably $C_2$-$C_{10}$, or a cyclic chemical structure. The molecular skeleton may be designed using a single molecule species, or alternatively using two or more molecule species. In an example of suitably applied embodiments where, for example, a detection-object molecule or the like is to be effectively detected, it is preferred that the thickness of the molecular mono-film (or molecular monolayer) formed by the binding species 7 is in the range of about 1.3 nm to 3 nm. In view of this, a binding species 7 having a $C_{11}$-$C_{20}$ alkane chain as a molecular skeleton is preferred. In such a case, for the long alkane chain immobilized on the surface of the metal fine-particle 3 via the functional group X2 extends vertically from the surface to form a molecular mono-film (molecular monolayer), the functional group Y2 suffuses the surface of the molecular mono-film (molecular monolayer). Well-known thiol compounds useful as reagents for forming self-assembly mono-films (SAM) can be suitably used as such binding species 11.

Because the nano-composite 10 of this embodiment includes metal fine-particles 3 immobilized in the matrix resin 1, and metal fine-particles 9 immobilized on the dottedly distributed portions of the metal fine-particles 3 exposed outside of the matrix resin 1 via the binding species 7, the relatively larger metal fine-particles 9 having an mean particle diameter greater than or equal to 40 nm can be formed in a state of substantially uniformly dispersed in a two-dimensional manner in the surface direction of the nano-composite 10. Therefore, as compared to the nano-composites having the metal fine-particles 3 only, the absorption spectrum of the LSPR is more intense and sharper. In view of this, with respect to the existence ratio of the metal fine-particles 3 having the exposed portions 3a to the metal fine-particles 9, it is preferred that the number of the metal fine-particles 3 having the exposed portions 3a is more than the number of the metal fine-particles 9 indirectly immobilized on the metal fine-particles 3 via the binding species 7. In order to obtain a sufficient LSPR effect, the existence ratio of the metal fine-particles 3 having the exposed portions 3a to the metal fine-particles 9, which is defined as the ratio of the number of the metal fine-particles 9 to the number of the metal fine-particles 3 having the exposed portions 3a, is preferably in the range of 0.01 to 1.0 and more preferably in the range of

0.02 to 1.0.

Moreover, the relationship between the mean particle diameter $D_{1A}$ of the metal fine-particles 3 and the mean particle diameter $D_{2A}$ of the metal fine-particles 9 is not particularly limited, but the relationship of "$D_{1A} \leq D_{2A}$" is preferred. Because the existence ratio of the metal fine-particles 9 to the metal fine-particles 3 gets smaller as the mean particle diameter $D_{2A}$ of the metal fine-particles 9 gets larger, in order to obtain a sufficient LSPR effect, the ratio ($D_{2A}/D_{1A}$) of the mean particle diameters $D_{1A}$ and $D_{2A}$ is preferably in the range of 4 to 20 and more preferably in the range of 5 to 15.

In the nano-composite 10 of this embodiment, the metal fine-particles 3 and the metal fine-particles 9 interact with light to induce LSPR. However, the absorption peak (referred to as "second peak" hereafter) of the LSPR caused by the interaction is at the longer wavelength side of the absorption peak (referred to as "first peak" hereafter) of the LSPR of the metal fine-particles 9 alone. For example, when the distance $L_2$ between a metal fine-particle 3 and a metal fine-particle 9 connected by the binding species 7, which is the shortest distance between the metal fine-particle 3 and the metal fine-particle 9 (not shown), is less than or equal to 3 nm, the second peak shifts toward the longer wavelength side (the wavelength region of 600 nm to 800 nm), and when $L_2$ exceeds 3 nm, the second peak is closer to the first peak. Therefore, the distance $L_2$ between a metal fine-particle 3 and a metal fine-particle 9 is preferably less than or equal to 3 nm.

The nano-composite 10 with the above constitution can be used in applications such as affinity bio-sensors, and so on. FIG. 4 is a schematic illustration of application of the nano-composite 10 as an affinity bio-sensor. At first, a nano-composite 10 is provided, which has a structure where metal fine-particles 9 are immobilized, via a binding species 7, on the exposed portions 3a of metal fine-particles 3 partially embedded in a matrix resin 1 and where another binding species 11 (a ligand) is bonded to the surfaces of the metal fine-particles 9, as also shown in FIG. 3. Next, a sample containing an analyte 30 and a non-detection object substance 40 is made contact with the nano-composite 10 having the binding species 11 being bonded to the metal fine-particles 9. Because the binding species 11 has a specific bindability with the analyte 30, a specific binding is produced between the analyte 30 and the binding species 11 through the contact. The non-detection object substance 40, which has no specific bindability with the binding species 11, does not bind with the binding species 11. As compared with the nano-composite 10 to which no analyte 30 but only the binding species 11 is bonded, the nano-composite 10 to which the analyte 30 is bonded via the binding species 11 has a change in the absorption spectrum of the LSPR, under light irradiation. That is, the developed color is changed. In this way, by detecting a change in the absorption spectrum of the LSPR, the analyte 30 in the sample can be detected with high sensitivity. The affinity bio-sensors utilizing LSPR does not need to use a label substance, and can be utilized as a technique for bio-sensors with simple constitutions in various fields.

<Fabrication Method>

Next, the method of fabricating the nano-composite 10 of this embodiment is described. The fabrication of the nano-composite 10 includes: 1) a step of forming a resin film containing a metal ion (or metal salt), 2) a reduction step, 3) an etching step, 4) a step of immobilizing the binding species 7, and 5) a step of immobilizing the metal fine-particles 9. Herein, a case where the matrix resin 1 constitutes a polyimide resin is described as a representative example.

1) The Step of Forming A Resin Film Containing A Metal Ion (or Metal Salt)

Firstly, a polyamic acid resin film (or polyamic acid resin layer) containing a metal ion (or metal salt) is prepared, possibly by using, for example, the casting method or the alkali modification described below.

Casting Method:

A casting method includes casting, on an arbitrary substrate, a polyamic acid resin solution containing a polyamic acid resin to form a polyamic acid resin film. Any of the following casting methods (I) to (III) can be used to form a polyamic acid resin film containing a metal ion (or metal salt):

(I) the method of casting a coating liquid containing a polyamic acid resin and a metal compound on an arbitrary substrate to form a polyamic acid resin film containing a metal ion (or metal salt);
(II) the method of casting a polyamic acid resin solution not containing a metal ion (or metal salt) on an arbitrary substrate to form a polyamic acid resin film, and then immersing the polyamic acid resin film in a solution containing a metal ion (or metal salt), which is referred to as "metal ion solution" hereafter; and
(III) the method of immersing, in a solution containing a metal ion (or metal salt), the polyamic acid resin film containing a metal ion (or metal salt) as formed with the above method (I).

The casting method is advantageous over the later-described alkali modification method, in certain aspects

including: easy control of the thickness of the metal fine-particle layer 5 or the matrix resin 1, easy application without a limitation on the chemical structure of the polyimide resin, and so on.

The merit of the above method (I) is that the amount of the metal compound contained in the polyamic acid resin solution can be easily adjusted. Thereby, for example, the amount of the metal contained in the nano-composite 10 can be easily adjusted, or a nano-composite 10 containing relatively larger metal fine-particles 3 with a particle diameter $D_1$ above 30 nm can be easily fabricated. That is, by using the above method (I), e.g., the particle diameter $D_1$ can be controlled in the range of 30 nm to 50 nm.

The merit of the above method (II) is that the metal ion (or metal salt) is impregnated in the polyamic acid resin film as being dissolved homogeneously and is thereby uniformly dispersed in the polyamic acid resin film with little variation. Hence, for example, a nano-composite 10 containing metal fine-particles 3 having a relatively narrower particle-diameter distribution can be fabricated.

The substrate used in the casting method is not particularly limited, in cases where the nano-composite 10 is peeled from the substrate to be used as a sensor or the like, or in cases where the nano-composite 10 utilizes the LSPR in a light-reflection manner while being attached with a substrate. In cases where the nano-composite 10 utilizes the LSPR in a light-transmission manner while being attached with a substrate, the substrate is preferably transparent to light, and, for example, a glass substrate or a transparent synthetic resin substrate, etc., can be used. Examples of the transparent synthetic resin include: polyimide resin, PET resin, acryl resin, MS resin, MBS resin, ABS resin, polycarbonate resin, silicone resin, siloxane resin, and epoxy resin, etc.

The polyamic acid resin as the precursor of a polyimide resin, which is referred to as "precursor" hereafter, can be a well-known polyamic acid resin obtained from well-known acid anhydride and diamine. The polyamic acid resin is obtained by, for example, dissolving in an organic solvent a tetracarboxylic dianhydride and a diamine in a substantially equimolar ratio, and stirring at a temperature in the range of 0-100°C for 30 min to 24 hours to conduct a polymerization reaction. Regarding the reaction, it is good to dissolve the reactants in a manner such that the amount of the obtained polyamic acid resin in the organic solvent is in the range of 5 wt% to 30 wt%, preferably in the range of 10 wt% to 20 wt%. The organic solvent used in the polymerization reaction may be a polar one. Examples of the organic polar solvent include: N,N-dimethylformamide, N,N-dimethylacetamide (DMAc), N-methyl-2-pyrrolidone, 2-butanone, dimethylsufoxide, dimethyl sulfate, cyclohexanone, dioxane, tetrahydrofuran, diglyme, and triglyme, etc. These solvents can be used in combination of two or more, and may also be used in part with an aromatic hydrocarbon such as xylene or toluene.

The synthesized polyamic acid resin is used in the form of a solution. It is usually advantageous to use a solution based on the reaction solvent, but, if required, the solution can be concentrated, diluted, or replaced by another organic solvent. A such adjusted solution can be utilized as a coating liquid by an addition of a metal compound.

The polyamic resin is preferably chosen such that the polyimide resin after the imidization has thermoplasticity and low thermal expandability. Moreover, the polyimide resin can be exemplified as a thermo-resistant resin constituted of a polymer having an imido group in its structure, such as polyimide, polyamideimide, polybenzimidazole, polyimide ester, polyetherimide, or polysiloxaneimide, etc.

Examples of the diamine suitably used to prepare the polyamic acid resin include 2,2'-bis(trifluoromethyl)-4,4'-diaminobiphenyl, 4,4'-diaminodiphenylether, 2'-methoxy-4,4'-diaminobenzanilide, 1,4-bis(4-aminophenoxy)benzene, 1,3-bis(4-aminophenoxy)benzene, 2,2-bis[4-(4-aminophenoxy)phenyl]propane, 2,2'-dimethyl-4,4'-diaminobiphenyl, 3,3'-dihydroxy-4,4'-diaminobiphenyl, and 4,4'-diaminobenzanilide, etc. Moreover, the diamine is suitably exemplified as 2,2-bis[4-(3-aminophenoxy)phenyl]propane, bis[4-(4-aminophenoxy)phenyl]sulfone, bis[4-(3-aminophenoxy)phenyl] sulfone, bis[4-(4-aminophenoxy)]biphenyl, bis[4-(3-aminophenoxy)biphenyl, bis[1-(4-aminophenoxy)]biphenyl, bis[1-(3-aminophenoxy)]biphenyl, bis[4-(4-aminophenoxy)phenyl]methane, bis[4-(3-aminophenoxy)phenyl]methane, bis[4-(4-aminophenoxy)phenyl]ether, bis[4-(3-aminophenoxy)phenyl]ether, bis[4-(4-aminophenoxy)]benzophenone, bis[4-(3-aminophenoxy)]benzophenone, bis[4,4'-(4-aminophenoxy)]benzanilide, bis[4,4'-(3-aminophenoxy)]benzanilide, 9,9-bis [4-(4-aminophenoxy)phenyl]fluorene, and 9,9-bis[4-(3-aminophenoxy)phenyl]fluorine, etc.

Examples of other amines include 2,2-bis[4-(4-aminophenoxy)phenyl]hexafluoropropane, 2,2-bis[4-(3-aminophenoxy)phenyl]hexafluoropropane, 4,4'-methylenedi-o-toluidine, 4,4'-methylenedi-2,6-xylidine, 4,4'-methylene-2,6-diethylaniline, 4,4'-diaminodiphenylpropane, 3,3'-diaminodiphenylpropane, 4,4'-diaminodiphenylethane, 3,3'-diaminodiphenylethane, 4,4'-diaminodiphenylmethane, 3,3'-diaminodiphenylmethane, 4,4'-diaminodiphenylsulfide, 3,3'-diaminodiphenylsulfide, 4,4'-diaminodiphenylsulfone, 3,3'-diaminodiphenylsulfone, 4,4'-diaminodiphenylether, 3,3-diaminodiphenylether, 3,4'-diaminodiphenylether, benzidine, 3,3'-diaminobiphenyl, 3,3'-dimethyl-4,4'-diaminobiphenyl, 3,3'-dimethoxybenzidine, 4,4"-diamino-*p*-terphenyl, 3,3"-diamino-*p*-terphenyl, *m*-phenylenediamine, *p*-phenylenediamine, 2,6-diaminopyridine, 1,4-bis(4-aminophenoxy)benzene, 1,3-bis(4-aminophenoxy)benzene, 4,4'-[1,4-phenylenebis(1-methylethylidene)]bisaniline, 4,4'-[1,3-phenylenebis(1-methylethylidene)]bisaniline, bis(*p*-aminocyclohexyl)methane, bis(p-$\beta$-amino-*t*-butylphenyl)ether, bis(*p*-$\beta$-methyl-$\delta$-aminopentyl)benzene, *p*-bis(2-methyl-4-aminopentyl)benzene, *p*-bis(1,1-dimethyl-5-aminopentyl)benzene, 1,5-diaminonaphthalene, 2,6-diaminonaphthalene, 2,4-bis($\beta$-amino-*t*-butyl) toluene, 2,4-diaminotoluene, *m*-xylene-2,5-diamine, *p*-xylene-2,5-diamine, *m*-xylylenediamine, *p*-xylylenediamine, 2,6-diaminopyridine, 2,5-diaminopyridine, 2,5-diamino-1,3,4-oxadiazole, and piperazine, etc.

The particularly preferred diamine component is exemplified as one or more diamines selected from 2,2'-bis (trifluoromethyl)-4,4'-diaminobiphenyl (TFMB), 1,3-bis(4-aminophenoxy)-2,2-dimethylpropane (DANPG), 2,2-bis[4-(4-aminophenoxy)phenyl]propane (BAPP), 1,3-bis(3-aminophenoxy)benzene (APB), paraphenylenediamine (*p*-PDA), 3,4'-diaminodiphenylether (DAPE34), and 4,4'-diaminodiphenylether (DAPE44).

Examples of the acid anhydride suitably used to prepare the polyamic acid resin include anhydrous pyromellitic acid, 3,3',4,4'-biphenyltetracarboxylic dianhydride, 3,3',4,4'-diphenylsulfonetetracarboxylic dianhydride, and 4,4'-oxydiphthalic anhydride. The followings are also preferred examples of the acid anhydride: 2,2',3,3'-, 2,3,3',4'-or 3,3',4,4'-benzophenonetetracarboxylic dianhydride, 2,3',3,4'-biphenyltetracarboxylic dianhydride, 2,2',3,3'-biphenyltetracarboxylic dianhydride, 2,3',3,4'-diphenylethertetracarboxylic dianhydride, and bis(2,3-dicarboxyphenyl)ether dianhydride, etc. Moreover, the followings are also preferred examples of the acid anhydride: 3,3",4,4"-, 2,3,3",4"- or 2,2",3,3"-*p*-terphenyltetracarboxylic dianhydride, 2,2-bis(2,3- or 3,4-dicarboxyphenyl)propane dianhydride, bis(2,3- or 3,4-dicarboxyphenyl)methane dianhydride, bis(2,3- or 3,4-dicarboxyphenyl)sulfone dianhydride, and 1,1-bis(2,3- or 3,4-dicarboxyphenyl) ethane dianhydride, etc.

The particularly preferred acid anhydride is exemplified as one or more acid anhydrides selected from anhydrous pyromellitic acid (PMDA), 3,3',4,4'-biphenyltetracarboxylic dianhydride (BPDA), 3,3',4,4'-benzophenonetetracarboxylic dianhydride (BTDA), and 3,3',4,4'-diphenylsulfonetetracarboxylic dianhydride (DSDA).

The diamines or the acid anhydrides can be used alone or in combination of two or more species. Moreover, diamines and acid anhydrides other than the above mentioned ones can also been used in combination.

In this embodiment, to prepare a coating liquid containing a metal compound or a polyamic acid resin solution not containing a metal ion (or metal salt), a commercially available solution containing a polyamic acid resin can be suitably used. Examples of the polyamic acid solution serving as a precursor of a thermoplastic polyimide resin include the thermoplastic polyamic acid resin varnishes SP1-200N™, SPI-300N™ and SPI-1000G™ produced by Nippon Steel Chemical Co., Ltd.), and Torayneece #3000™ produced by Toray Industries, Inc., etc. Moreover, examples of the polyamic acid solution serving as a precursor of a non-thermoplastic polyimide resin include the non-thermoplastic polyamic acid resin varnishes U-varnish-A™ and U-varnish-S™ produced by UBE Industries, Ltd., etc.

When the nano-composite 10 is suitably used in an application utilizing LSPR of the light transmission type, for example, it is preferred to use a transparent or colorless polyimide resin, wherein an intra- or inter-molecular charge-transfer (CT) complex is difficult to form, such as an aromatic polyimide resin having a substituent with a bulky steric structure, an alicyclic polyimide resin, a fluoro-polyimide resin, or a silicon-polyimide resin, etc.

The above substituent with a bulky steric structure may have, for example, a fluorene skeleton or an admantane skeleton, etc. In the aromatic polyimide resin, such a substituent with a bulky steric structure may be located on any one of the acid anhydride residue and the diamine residue, or on both of them. A diamine having a substituent with a bulky steric structure can be exemplified by 9,9-bis(4-aminophenyl)fluorene, etc.

The alicyclic polyimide resin is a resin formed by polymerizing an alicyclic acid anhydride and an alicyclic diamine. Moreover, the alicyclic polyimide resin can also be obtained by hydrogenating an aromatic polyimide resin.

The fluoro-polyimide resin is a resin formed by polymerizing an acid anhydride and/or a diamine in which a monovalent element bonded to a carbon of, for example, alkyl or phenyl, etc., is substituted by fluorine, a perfluoroalkyl group, a perfluoroaryl group, a perfluoroalkoxy group, or a perfluorophenoxy group, etc. Though the monovalent element can be totally or partially substituted by fluorine atoms, it is preferred that 50% or more of the monovalent element is substituted.

The silicon-polyimide resin is a resin obtained by polymerizing a silicon-containing diamine and an acid anhydride.

It is preferred that such a transparent polyimide resin, for example at a thickness of 10 $\mu$m, has a transmittance greater than or equal to 80% at the wavelength 400 nm, and a mean transmittance of visible light greater than or equal to 90%.

Among the above polyimide resins, the fluoro-polyimide resin with a particularly high transparency is preferred. A polyimide resin with a constituting unit expressed by general formula (1) can be used as the fluoro-polyimide resin. Herein, in general formula (1), $Ar_1$ is a tetravalent aromatic group expressed by formula (2), (3) or (4), $Ar_2$ is a divalent aromatic group expressed by formula (5), (6), (7) or (8), and p is the repetition number of the constituting unit.

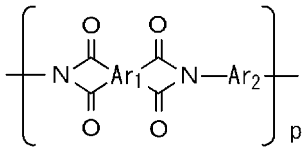

· · · (1)

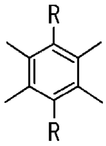 · · · (2) 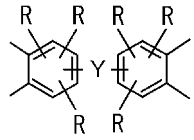 · · · (3)

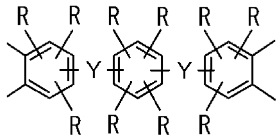 · · · (4)

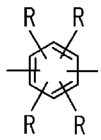 · · · (5) 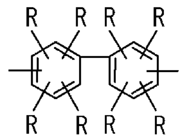 · · · (6) 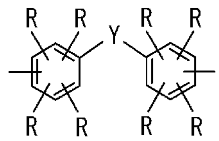 · · · (7)

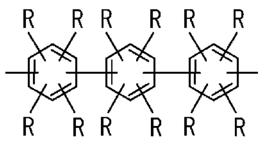 · · · (8)

Moreover, each R is independently a fluorine atom or a perfluoroalkyl group, Y is a divalent group expressed by the following structural formulae, $R_1$ is a perfluoroalkylene group, and n is a number of 1 to 19.

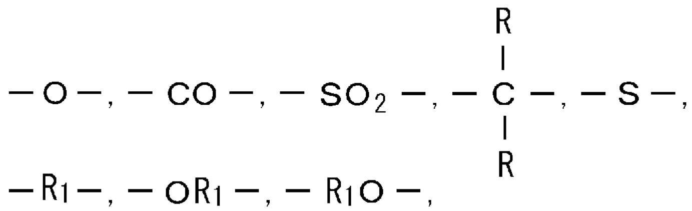

$-(OR_1)_n-$, $-(R_1O)_n-$, $-(OR_1O)_n-$

or

$-O-CO-R_1-CO-O-$

Because in the above general formula (1) $Ar_2$ can be deemed an amine residue and $Ar_1$ be deemed an acid anhydride residue, the preferred fluoro-polyimide resin is described by exemplifying the diamine, and the acid anhydride or a tetracarboxylic acid, an acid chloride or an esterfied compound that can be utilized equivalently (referred to as "acid anhydride, etc." hereafter). However, the fluoro-polyimide resin is not limited to be obtained from the diamines and the acid anhydrides, etc., described herein.

The raw-material diamine corresponding to $Ar_2$ can be any amine if only any monovalent element bonded to the carbon of an alkyl group or a phenyl ring, etc., other than the amino group in the molecule, is fluorine or a perfluoroalkyl group. Such diamine can be exemplified as 3,4,5,6,-tetrafluoro-1,2-phenylenediamine, 2,4,5,6-tetrafluoro-1,3-phenylenediamine, 2,3,5,6-tetrafluoro-1,4-phenylenediamine, 4,4'-diaminooctafluorobiphenyl, bis(2,3,5,6-tetrafluoro-4-aminophenyl)ether, bis(2,3,5,6-tetrafluoro-4-aminophenyl)sulfone, hexafluoro-2,2'-bistrifluoromethyl-4,4'-diaminobiphenyl, or 2,2-bis(trifluoromethyl)-4,4'-diaminobiphenyl, etc.

Examples of the raw-material acid anhydride corresponding to $Ar_1$ include 1,4-difluoropyromellitic acid, 1-trifluoromethyl-4-fluoropyromellitic acid, 1,4-di(trifluoromethyl)pyromellitic acid, 1,4-di(pentafluoroethyl)pyromellitic acid, hexafluoro-3,3',4,4'-bisphenyltetracarboxylic acid, hexafluoro-3,3',4,4'-benzophenonetetracarboxylic acid, 2,2-bis(3,4-dicarboxytrifluorophenyl)hexafluoropropane, 1,3-bis(3,4'-dicarboxytrifluorophenyl)hexafluoropropane, 1,4-bis(3,4-dicarboxytrifluorophenoxy)tetrafluorobenzene, hexafluoro-3,3',4,4'-oxybisphthalic acid, and 4,4'-(hexafluoroisopropylidene)diphthalic acid, etc.

As the metal compound contained in the coating liquid together with the polyamic acid resin prepared for the above method (I), or the metal compound contained in the solution containing a metal ion (or metal salt) prepared for the above method (II), a compound that contains the above metal species constituting the metal fine-particles 3 can be used without a particular limitation. As the metal compound, a salt or an organic carbonyl complex, etc., of the above metal can be used. The metal salt can be exemplified as a hydrochloric salt, a sulfate salt, an acetate salt, an oxalate salt, or a citrate salt, etc. Moreover, the organic carbonyl compound obtained by forming an organic carbonyl complex from the above metal species can be exemplified as acetylacetone, benzoylacetone, a $\beta$-diketone such as dibenzoylmethane, or a $\beta$-ketocarboxylate ester such as ethyl acetoacetate, etc.

Preferred specific examples of the metal compound may include: $H[AuCl_{4]}$, $Na[AuCl_4]$, AuI, AuCl, $AuCl_3$, $AuBr_3$, $NH_{4[}AuCl_4]\cdot n2H_2O$, $Ag(CH_3COO)$, AgCl, $AgC10_4$, $Ag_2CO_3$, AgI, $Ag_2SO_4$, $AgNO_3$, $Ni(CH_3COO)_2$, $Cu(CH_3COO)_2$, $CuSO_4$, $CuSO_4$, $CuSO_4$, $CuCl_2$, $CuSO_4$, $CuBr_2$, $Cu(NH_4)_2Cl_4$, CuI, $Cu(NO_3)_2$, $Cu(CH_3COCH_2COCH_3)_2$, $CoCl_2$, $CoCO_3$, $CoSO_4$, $Co(NO_3)_2$, $NiSO_4$, $NiCO_3$, $NiCl_2$, $NiBr_2$, $Ni(NO_3)_2$, $NiC_2O_4$, $Ni(H_2PO_2)_2$, $Ni(CH_3COCH_2COCH_3)_2$, $Pd(CH_3COO)_2$, $PdSO_4$, $PdCO_3$, $PdCl_2$, $PdBr_2$, $Pd(NO_3)_2$, $Pd(CH_3COCH_2COCH_3)_2$, $SnCl_2$, $IrCl_3$, and $RhCl_3$, etc.

In the coating liquid containing a polyamic acid resin prepared for the above method (1) and a metal compound, the metal ion formed by dissociation of the metal compound may cause a three-dimensional crosslinking reaction between the polyamic acid resin, depending on the species of the metal. Hence, the coating liquid may be thickened/gelated as time goes on and may be difficult to use. To prevent such thickening/gelation, it is preferred to add in the coating liquid a viscosity adjusting agent as a stabilizing agent. By adding the viscosity adjusting agent, the metal ion in the coating liquid forms a chelate complex with the viscosity adjusting agent instead but not with the polyamic acid resin. In this way, three-dimensional crosslinking between the polyamic acid resin and the metal ion is blocked by the viscosity adjusting agent, and the thickening/gelation is inhibited.

As the viscosity adjusting agent, it is preferred to select a low-molecular organic compound that has a high reactivity with the metal ion, i.e., allows the formation of a metal complex. The molecular weight of the low-molecular organic compound is preferably within the range of 50 to 300. Specific examples of such viscosity adjusting agents may include acetylacetone, ethyl acetoacetate, pyridine, imidazole, and picoline, etc. Moreover, the addition amount of the viscosity adjusting agent relative to 1 mole of the formed chelate complex compound is preferably in the range of 1 mole to 50 moles and more preferably in the range of 2 moles to 20 moles.

The amount of the metal compound in the coating liquid, relative to 100 parts by weight of the combination of the polyamic acid resin, the metal compound and the viscosity adjusting agent, is made in the range of 3 wt% to 80 wt% and preferably in the range of 20 wt% to 60 wt%. If the amount of the metal compound is less than 3 parts by weight, separation of the metal fine-particles 3 is insufficient. If the amount exceeds 80 parts by weight, the metal salt that cannot be dissolved in the coating liquid precipitates, so that the metal fine-particles 3 tend to aggregate easily.

Moreover, in the coating liquid, arbitrary component other than the above ones, such as a leveling agent, an anti-foaming agent, an adhesion promoter or a crosslinking agent, etc., may be added.

The method of coating the coating liquid containing a metal compound or the polyamic resin solution not containing a metal ion (or metal salt) is not particularly limited, and is possibly a coating method using a coater such as a comma, a die, a knife or a lip, etc. However, among the coaters, the preferred ones are spin coater, gravure coater, and bar coater, etc., that allows the formation of a uniform coated film (or polyamic acid resin film) to easily control the thickness of the matrix resin 1 in a high precision.

Moreover, in the metal ion solution used in the above method (II), the metal compound in contained preferably in the range of 30 mM to 300 mM, more preferably in the range of 50 mM to 100 mM. If the concentration of the metal compound is less than 30 mM, the time required to impregnating the metal ion solution in the polyamic acid resin film is overly long, which is not preferred. If the concentration exceeds 300 mM, it is worried that the surface of the polyamic acid resin film is eroded (dissolved).

In addition to the metal compound, the metal ion solution can also contain a component for adjusting the pH value, such as a buffer solution, etc.

The impregnation method is not particularly limited as long as it makes the surface of the polyamic acid resin film contact with the metal ion solution, and can be a well known method, such as a dipping method, a spray method, a brush coating method or a printing method, etc. The impregnation temperature is within the range of 0-100°C, and is preferably a normal temperature around 20-40°C. Moreover, when the dipping method is applied, the impregnation time is, for example, preferably from 1 min to 5 hours and more preferably from 5 minutes to 2 hours. If the impregnation time is less than 1 min, impregnation of the metal ion solution in the polyamic acid resin film is insufficient. On the contrary, if the impregnation time exceeds 5 hours, the degree of the impregnation of the metal ion solution in the polyamic acid resin film tends to be unvaried substantially.

After the coating liquid containing a metal compound or the polyamic acid resin solution not containing a metal ion (or metal salt) is coated, drying is conducted to form a polyamic acid resin film. During the drying, the temperature is controlled such that the imidization caused by dehydration and ring closure of the polyamic acid resin is not completed.

The drying method is not particularly limited, and the drying may be conducted, for example, at a temperature in the range of 60-200°C for 1 min to 60 min, and preferably at a temperature in the range of 60-150°C. It does not matter if a part of the structure of the polyamic acid resin in the dried polyamic acid resin film is imidized. However, it is feasible that the imidization ratio is 50% or less and more preferably 20% or less and 50% or more of the polyamic acid structure remains. Moreover, the imidization ratio of the polyamic acid resin can be calculated, while a Fourier-Transform IR spectroscope (a commercially available product is, for example, FT/IR620 manufactured by JASCO Corporation) is used to measure the IR absorption spectrum of the film with a transmission-type method, from the absorbance at 1710 $cm^{-1}$ due to the imide group, with the absorbance at 1000 $cm^{-1}$ due to the C-H bond on the benzene ring as a reference.

The polyamic acid resin film may be a single layer, or a laminated structure formed from a plurality of polyamic acid resin films. In the case of multiple layers, the film can be formed by coating, on layers of polyamic acid resins having different constituting components, other polyamic acid resin(s) in sequence. When the polyamic acid resin film includes 3 or more layers, the polyamic acid resin of the same constitution may be used 2 or more times. When the film has a simple 2-layer or single-layer structure, especially a single-layer structure, it is beneficial in the industry.

Moreover, after a single layer or multiple layers of polyamic acid resins are laminated on a sheet-like support member and then imidized to form a single layer or multiple layers of polyimide resins, it is also possible to further form a polyamic acid resin film on the same. In such a case, to promote the adhesion between the polyimide resin layer and the polyamic acid resin film, the surface of the polyimide resin layer is preferably surface-treated by plasma. With such plasma surface treatment, the surface of the polyimide resin layer can be roughened, or the chemical structure of the surface is altered. Thereby, the wettability of the surface of the polyimide resin layer, the affinity thereof with the polyamic acid resin solution is raised, and the polyamic acid resin film can be stably maintained on the surface.

Alkali Modification Method

The alkali modification method includes modifying the surface of a polyimide film by an alkali to form a polyamic acid resin layer and then impregnating a metal ion solution in the polyamic acid resin layer. The used polyimide resin is the same as that used in the above casting method, so its description is omitted.

Because the metal ion (or metal salt) is impregnated in the polyamic acid resin layer in a state of being homogeneously dissolved in the metal ion solution and thereby uniformly dispersed in the polyamic acid resin layer with little concentration variation, the alkali modification has, for example, the following merits etc. A nano-composite 10 containing metal fine-particles 3 with a relatively narrower particle-diameter distribution can be made. An integral-type nano-composite 10 having a high adhesion with a polyimide film substrate can be made. When a nano-composite 10 is being made at the surface side of a polyimide film, the same process can be conducted at the back side of the polyimide film simultaneously. Or, the metal ion in the metal ion solution can be easily ion-exchanged with the salt of the alkali metal and the carboxylate groups at the terminals of the polyimide chains caused by the aqueous alkali solution, so that the impregnation time can be shortened.

The aqueous alkali solution for treating the polyimide film is preferably an aqueous solution of sodium hydroxide or potassium hydroxide having a concentration in the range of 0.5 wt% to 50 wt% and a liquid temperature in the range of 5-80°C. The aqueous alkali solution can be suitably applied with a dipping method, a spray method or brush coating method, etc. In a case using the dipping method, for example, it is effective to treat the polyimide film by the aqueous alkali solution for 10 sec to 60 min. It is preferred that the surface of the polyimide film is treated, by an aqueous alkali solution with a concentration in the range of 1 wt% to 30 wt% and a liquid temperature in the range of 25-60°C, for 30 sec to 10 min. The treatment condition can be suitably changed depending on the structure of the polyimide film. In general, when the concentration of the aqueous alkali solution is lower, the treatment time of the polyimide film becomes longer. Moreover, when the liquid temperature of the aqueous alkali solution is higher, the treatment time is shortened.

By the treatment with the aqueous alkali solution, the aqueous alkali solution penetrates from the surface side of the polyimide film to modify the polyimide resin. The modification reaction caused by the alkali treatment is considered to be based on hydrolysis of the imido bonds. The thickness of the alkali-treated layer formed by the alkali treatment is preferably in the range of 1/5000 to 1/2, more preferably in the range of 1/3000 to 1/5, of the thickness of the polyimide film. In another view point, the thickness of the alkali-treated layer is suitably in the range of 0.005 $\mu$m to 3.0 $\mu$m, preferably in the range of 0.05 $\mu$m to 2.0 $\mu$m, and more preferably in the range of 0.1 $\mu$m to 1.0 $\mu$m. By setting the thickness in such range, the formation of the metal fine-particles 3 is facilitated. If the thickness of the alkali-treated layer is less than the lower limit (0.005 $\mu$m), it is difficult to sufficiently impregnate the metal ion. On the other hand, in the treatment of the polyimide resin with the aqueous alkali solution, the outmost portion of the polyimide resin tends to be dissolved simultaneously when the imido-ring of the polyimide resin is opened, so that the above upper limit (3.0 $\mu$m) is difficult to exceed. Moreover, when the metal fine-particles 3 are to be substantially dispersed two-dimensionally, the thickness of the alkali-treated layer formed by the alkali treatment is preferably in the range of 1/5000 to 1/20, more preferably in the range of 1/500 to 1/50, of the thickness of the polyimide film. In another view point, the thickness of the alkali-treated layer is suitably in the range of 20 nm to 150 nm, preferably in the range of 50 nm to 150 nm, and more

preferably in the range of 100 nm to 120 nm. By setting the thickness in such range, the formation of the metal fine-particles 3 is facilitated.

In view of the ease of the modification of the polyimide film with the aqueous alkali solution, it is desired to select a polyimide film having a high water absorption ratio. The water absorption ratio of the polyimide film is preferably 0.1 % or more, and more preferably 0.2% or more. If the water absorption ratio is less than 0.1%, the modification is insufficient, or the modification time is required to be well increased, which is not good.

Moreover, because the degree of the modification treatment of the polyimide film by the aqueous alkali solution varies when the chemical structure of the polyimide resin constituting the polyimide film is different, it is preferred to select a polyimide film easily subjected to the modification treatment. Examples of such polyimide film suitably subjected to a modification treatment with an aqueous alkali solution include: a polyimide film having ester bonds in its structure, and a polyimide film using anhydrous pyromellitic acid as one of the acid anhydride monomers. The amount of the anhydrous pyromellitic acid relative to 100 moles of the acid anhydride component is preferably 50 moles or more and more preferably 60 moles or more.

It is also feasible to simultaneously modify both sides of the polyimide film with the alkali treatment, depending on the application. A polyimide resin layer constituted of a polyimide resin with a low thermal expandability, to which the alkali treatment is particularly effective, is suitable. A polyimide resin with a low thermal expandability has a good familiarity (wettability) with respect to the aqueous alkali solution, so that the ring opening reaction of the imide ring is easily induced.

In the alkali-treated layer, a salt of the alkali metal from the aqueous alkali solution and the terminal carboxyl groups of the polyimide resin, etc., are formed. The alkali metal carboxylate salt of the carboxyl can be substituted by the salt of the metal ion through the metal ion solution impregnation treatment in the subsequent metal ion solution impregnation step, so there is no problem even if the salt of the metal ion exists before the subsequent metal ion solution impregnation step is conducted. Moreover, the surface layer of the polyimide resin turned to be alkaline may be neutralized by an aqueous acid solution. The aqueous acid solution can be arbitrary aqueous solution as long as it is acidic, but is particularly preferably an aqueous hydrochloric acid solution or an aqueous sulfuric acid solution. Moreover, the concentration of the aqueous acid solution is suitably in the range of 0.5 wt% to 50 wt%, preferably in the range of 0.5 wt% to 5 wt%. It is more preferred that the pH value of the aqueous acid solution is 2 or less. After being cleaned by the aqueous acid solution, washed by water and then dried, the treated polyimide film is provided to the subsequent metal ion solution impregnation step.

The polyimide film being formed with an alkali-modified layer is subjected to the impregnation of a metal ion solution and then dried to form a layer containing a metal ion (or metal salt). With the impregnation treatment, the carboxyl group present in the alkali-modified layer is made into a metal carboxylate salt.

The metal ion and the metal compound, and the metal ion solution used in the impregnation step can be the same as those used in the above casting method.

The impregnation method is not particularly limited as long as the metal ion solution is able to contact the surface of the alkali-modified layer, and can be a well known method. For example, a dipping method, a spray method, a brush coating method or a printing method, etc., can be used. The impregnation temperature is in the range of 0-100°C, and is preferably a room temperature around 20-40°C. Moreover, when the dipping method is used, the impregnation time is, for example, preferably 1 min to 5 hours and more preferably 5 min to 2 hours.

The film is dried after the impregnation. The drying method is not particularly limited, and may be, for example, natural drying, blow-drying using an air gun, or drying with an oven, etc. The drying condition is 10-150°C for 5 sec to 60 min, preferably 25-150°C for 10 sec to 30 min, and more preferably 30-120°C for 1-10 min.

In "the polyamic acid resin film or the polyamic acid resin layer containing a metal ion or a metal salt" (referred to as "a polyamic acid resin layer containing a metal ion" hereafter) formed by the above casting method or alkali modification method, the metal ion is adsorbed on the carboxyl group due to the interaction between the metal ion and the carboxyl group of the polyamic acid resin, and a complex is formed. Such a phenomenon has an effect of uniformizing the concentration distribution of the metal ion in the polyamic acid resin layer containing a metal ion. Hence, there is an effect that an uneven distribution or aggregation of the metal fine-particles 3 separated from the matrix resin 1 is prevented, and metal fine-particles 3 having a uniform shape are separated from the resin in a uniform distribution.

(2) Reduction Step:

In the reduction step, the above obtained polyamic acid resin layer containing a metal ion is thermally treated to reduce the metal ion (or metal salt) and separate metal fine-particles 3, preferably at a temperature of 140°C or higher, more preferably at a temperature in the range of 160-450°C and even more preferably in the range of 200-400°C. If the temperature of the thermal treatment is lower than 140°C, the reduction of the metal ion (or metal salt) is insufficient, and it is difficult to make the mean particle diameter $D_{1A}$ of the metal fine-particles 3 greater than or equal to the lower limit (3 nm). Moreover, if the temperature of the thermal treatment is lower than 140°C, in the matrix resin 1, thermal diffusion of the metal fine-particles 3 being separated due to the reduction is not induced sufficiently. Further, if the

temperature of the thermal treatment is lower than 140°C, in cases where a polyimide resin is used as the matrix resin 1, the imidization of the precursor of the polyimide resin is insufficient, and a re-heating step for imidization is required. On the contrary, if the temperature of the thermal treatment is above 450°C, the matrix resin 1 is decomposed thermally, so that new absorption due to the decomposition of the matrix resin 1 is easily produced in addition to the absorption due to the LSPR, or the distance between neighboring metal fine-particles 3 becomes small to easily cause an interaction between neighboring metal fine-particles 3, etc. Therefore, the absorption spectrum due to the LSPR is broadened. Moreover, the time of the thermal treatment can be determined according to the target inter-particle distance and further according to the temperature of the thermal treatment or the amount of the metal ion (or metal salt) contained in the polyamic acid layer. For example, the time can be set in the range of 10 min to 180 min when the temperature of the thermal treatment is 200°C, or in the range of 1 min to 60 min when the temperature of the thermal treatment is 400°C.

The particle diameter $D_1$ and the inter-particle distance $L_1$ of the metal fine-particles 3 can be controlled based in the heating temperature and the heating time in the reduction step, and the amount of the metal ion (or metal salt) contained in the matrix resin 1 or its precursor. The inventors have discovered that when the heating temperature and the heating time in the thermal reduction are fixed, the particle diameter $D_1$ of the separated metal fine-particles 3 is changed if the absolute amount of the metal ion (or metal salt) contained in the matrix resin 1 or its precursor is changed. Moreover, there was also a discovery that when the thermal reduction is conducted without controlling the heating temperature and the heating time, the inter-particle distance $L_1$ becomes smaller than the particle diameter $D_{1L}$ of the larger one of neighboring metal fine-particles 3, or the metal fine-particles 3 aggregate on the surface of the matrix resin 1 to form island structures.

In view of the above discoveries, it is known that the particle diameter $D_1$ of the metal fine-particles can be controlled by controlling the heating temperature, and the inter-particle distance $L_1$ can be controlled by controlling the heating time. These aspects will be specifically described later in the example. For example, when gold ion contained in an amount of 18 wt% per unit volume ($cm^3$) [5.2 $\mu$g per unit area ($cm^2$)] in a polyamic acid resin (a precursor resin of the matrix resin 1) of 200 nm thick is thermally reduced in the atmosphere, the particle diameter $D_1$ and the inter-particle distance $L_1$ of the metal fine-particles formed through the thermal reduction are varied according to the heating temperature and the heating time. Specifically, the particle diameter $D_1$ is about 9 nm (the mean particle diameter $D_{1A}$ is about 9 nm) by a treatment at 200°C for 10 min, is about 13 nm (the mean particle diameter $D_{1A}$ is about 13 nm) by a treatment at 300°C for 3 min, or is about 15 nm (the mean particle diameter $D_{1A}$ is about 15 nm) by a treatment at 400°C for 1 min. In any of the cases, the nano-composite is formed in a manner such that the distance between any pair of neighboring metal gold fine-particles is greater than or equal to the particle diameter $D_{1L}$ of the larger one of the pair of neighboring metal gold fine-particles, or is approximately close to the particle diameter $D_{1L}$. Based on such an example, by further controlling the thickness of the metal fine-particle layer 5 in the above range, a nano-composite 10 meeting the above requirements can be formed.

Moreover, based on the above discoveries, for example, the thermal treatment in the reduction step can be conducted in multiple stages. For example, it is possible to conduct a particle-diameter controlling step that grows the metal fine-particles 3 to a predetermined particle diameter $D_1$ at a first heating temperature, and an inter-particle distance controlling step that maintains the inter-particle distance $L_1$ of the metal fine-particles 3 in a predetermined range at a second heating temperature being the same as or different from the first heating temperature. In this way, by adjusting the first and the second heating temperatures and the heating time, the particle diameter $D_1$ and the inter-particle distance $L_1$ can be controlled more precisely.

The reasons that thermal reduction is adopted as the reduction method include industrial merits, such as, that the particle diameter $D_1$ and the inter-particle distance $L_1$ can be controlled more easily by controlling the treatment condition (especially the heating temperature and the heating time), that it can be coped with a simple equipment without a particular limitation from the lab scale to the production scale, and that it can be coped with in a single-piece manner or a continuous manner without special efforts, etc. The thermal reduction can be conducted, for example, in an atmosphere of an inert gas such as Ar or $N_2$ etc., in a vacuum of 1-5 kPa, or in the atmosphere. The reduction method is not suitably a vapor reduction method using a reductive gas such as hydrogen, or a light reduction method. In vapor reduction, metal fine-particles 3 are not present near the surface of the matrix resin 1, and thermal decomposition of the matrix resin 1 is enhanced by the reductive gas, so the inter-particle distance of the metal fine-particles 3 is difficult to control. Moreover, in light reduction, a variation in the density of the metal fine-particles 3 from near the surface to the deeper portion of the matrix resin 1 is easily caused by the light transparency of the matrix resin 1; so not only the particle diameter $D_1$ and the inter-particle distance $L_1$ of the metal fine-particles 3 are difficult to control, but also and the reduction efficiency is low.

In the reduction step, the imidization of the polyamic acid resin can be completed by utilizing the heat utilized in the reduction treatment, so that the process from the separation of the metal fine-particles 3 to the imidization can be conducted in one pot, and the manufacturing process can be simplified.

In the thermal reduction, the metal ion (or metal salt) existing in the matrix resin 1 or its precursor is reduced, and respective metal fine-particles 3 can be separated independently due to thermal diffusion. Such formed metal fine-particles 3 are in a state that the inter-particle distance $L_1$ is kept equal to or larger than a predetermined value, and also have an approximately uniform shape, while the metal fine-particles 3 are not unevenly dispersed in the matrix resin 1. Particularly, when the metal ion (or metal salt) in the polyamic acid resin layer containing a metal ion is adsorbed by the carboxyl groups of the polyamic acid resin to form a complex, an intermediate of the nano-composite, in which the shape and the particle diameter $D_1$ of the metal fine-particles 3 are uniformized and eventually the metal fine-particles 3 are uniformly separated and dispersed in the matrix resin 1 in an approximately uniform inter-particle distance $L_1$, can be obtained. Moreover, the particle diameter $D_1$ and the distribution state of the metal fine-particles 3 in the matrix resin 1 can be controlled by controlling the constituting unit of the resin constituting the matrix resin 1, or by controlling the absolute amount of the metal ion (or metal salt) and the volume fraction of the metal fine-particles 3.

(3) Etching Step:

In the etching step, a part of the metal fine-particles 3 existing in the matrix resin 1 are exposed out of the surface of the matrix resin 1. The etching step is conducted by, for example, etching away a surface layer of the matrix resin 1 in the intermediate of the nano-composite that is at the side where the metal fine-particles 3 are to be exposed. The etching method is, for example, a wet etching method using a hydrazide-series solution or an alkali solution, or a dry etching method utilizing a plasma treatment.

Regarding the wet etching method, as a matrix resin 1 that can be suitably etched by, for example, an alkali solution, it is desired to select a resin having a high water absorption ratio, in view of the ease of the penetration of the etching solution. The water absorption ratio is preferably 0.1 % or more and more preferably 0.2% or more.

Regarding the dry etching method, as a matrix resin 1 that can be suitably etched by, for example, plasma, it is desired to select a resin having a polar group such as -OH, -SH, -O-, -S-, -SO-, -NH-, -CO-, -CN, -P=O, -PO-, $-SO_2-$ -CONH- or $-SO_3H$, etc., in view of the high reactivity with the gas in the plasma state. Moreover, in another viewpoint, as in the case were the etching utilizes an alkali solution, it is desired to select a matrix resin 1 having a high water absorption ratio, which is preferably 0.1 % or more and more preferably 0.2% or more.

(4) Step of Immobilizing the Binding Species:

In the step of immobilizing the binding species 7, the binding species 7 is immobilized on the surfaces of the portions 3a of the metal fine-particles 3 exposed outside of the matrix resin 1. The step of immobilizing the binding species 7 can be conducted by having the binding species 7 to contact with the surfaces of the exposed portions 3a of the metal fine-particles 3. For example, it is preferably to conduct a surface treatment of the metal fine-particles 3 using a treating liquid obtained by dissolving the binding species 7 in a solvent. The solvent for dissolving the binding species 7 can use, but is not limited thereto, water, $C_1$-$C_8$ hydrocarbon alcohols such as methanol, ethanol, propanol, isopropanol, butanol, *t*-butanol, pentanol, hexanol, heptanol and octanol, etc., $C_3$-$C_6$ hydrocarbon ketones such as acetone, propanone, methylethyl ketone, pentanone, hexanone, methylisobutyl ketone and cyclohexanone, etc., $C_4$-$C_{12}$ hydrocarbon ethers such as diethylether, ethylene glycol dimethylether, diethylene glycol dimethylether, diethylene glycol diethylether, diethylene glycol dibutylether and tetrahydrofuran, etc., $C_3$-$C_7$ hydrocarbon esters such as methyl acetate, ethyl acetate, propyl acetate, butyl acetate, γ-butyrolactone and diethyl malonate, etc., $C_3$-$C_6$ amides such as dimethylformamide, dimethylacetoamide, tetramethylurea and hexamethylphosphoric triamide, etc., $C_2$ sulfoxides such as dimethylsulfoxide, etc., $C_1$-$C_6$ halogen-containing compounds such as chloromethane, bromomethane, dichloromethane, chloroform, carbon tetrachloride, dichloroethane, 1,2-dichloroethane, 1,4-dichlorobutane, trichloroethane, chlorobenzene and o-dichlorobenzene, etc., and $C_4$-$C_8$ hydrocarbon compounds such as butane, hexane, heptane, octane, benzene, toluene and xylene, etc.

The concentration of the binding species 7 in the treating liquid is preferably from 0.0001 M (mol/L) to 1 M, for example. Although a low concentration leads to a merit that a small amount of remaining binding species 7 is bonded to the surface of the metal fine-particles 3, the concentration is more preferably from 0.005 M to 0.05 M.

In a case where the surfaces of the metal fine-particles 3 are to be treated by the above treating liquid, the treatment method is not limited as long as the treating liquid can contact the exposed portions 3a of the metal fine-particles 3, while a uniform contact is preferred. For example, the metal fine-particles 3 having the exposed portions 3a may be immersed in the treating liquid together with the matrix resin 1, or the treating liquid may be sprinkled to the exposed portions 3a of the metal fine-particles 3 in the matrix resin 1 by spraying, etc., or the treating liquid may be coated using a suitable tool. Moreover, the temperature of the treating liquid at this moment is not particularly limited, and is preferably 20°C or lower, more preferably in the range of -20°C to 20°C and desirably in the range of -10°C to 20°C. By setting the temperature of the treating liquid to be 20°C or lower, the direct bonding of the binding species 7 to the matrix resin 1 is inhibited, and the binding species 7 can be selectively bonded to the exposed portions 3a of the metal fine-particles 3. Hence, by setting the temperature of the treating liquid to be 20°C or lower, the metal fine-particles 9 are prevented from being eventually immobilized on the surface of the matrix resin 1 via the binding species 7 and forming aggregates

to degrade the LSPR effect. Moreover, when the surface treatment adopts the immersion method, for example, the immersion time is preferably from 1 min to 24 hours.

After the surface treatment is finished, it is preferred to conduct a cleaning step that uses an organic solvent to dissolve and remove the excess binding species 7 adhering to the surfaces of the metal fine-particles 3. The organic solvent used in the cleaning step can be one capable of dissolving the binding species 7. Examples of the solvents can be the above exemplified solvents for dissolving the binding species 7.

In the cleaning step, the method of cleaning the surfaces of the metal fine-particles 3 by the organic solvent is not limited. It is possible to immerse the metal fine-particles 3 in the organic solvent, to sprinkle the organic solvent by spraying, etc., to flush the metal fine-particles 3, or to soak a suitable material in the organic solvent and wipe the metal fine-particles 3 with the material. In this cleaning step, though the excess binding species 7 adhering to the surfaces of the metal fine-particles 3 is dissolved and removed, the binding species 7 is not entirely removed. It is advantageous that the binding species 7 is removed by the cleaning to an extent that the film of the binding species 7 on the surfaces of the metal fine-particles 3 has approximately the thickness of a molecular mono-film. In a method to achieve this, a water cleaning step is conducted before the above cleaning step, the above cleaning step is conducted, and then another water cleaning step is conducted. The temperature of the organic solvent in the above cleaning step at this moment is preferably from 0°C to 100°C and more preferably in the range of 5-50°C. Moreover, the cleaning time is preferably from 1 sec to 1000 sec and more preferably in the range of 3 sec to 600 sec. The amount of the organic solvent used is preferably from 1 L to 500 L, more preferably in the range of 3 L to 50 L, per 1 $m^2$ of the surface area of the nano-composite 10.

Moreover, if required, it is preferred to remove the binding species 7 adhering to the surface of the matrix resin 1 by an alkali solution. The alkali solution used at this moment preferably has a concentration from 10 mM (mmol/L) to 500 mM and a temperature from 0°C to 50°C. When the alkali solution is used for immersion, for example, the immersion time is preferably from 5 sec to 3 min.

(5) Step of Immobilizing the Metal Fine-Particles 9:

In the step of immobilizing the metal fine-particles 9, the metal fine-particles 9 are bonded to the binding species 7 and indirectly immobilized on the metal fine-particles 3 via the binding species 7. The step of immobilizing the metal fine-particles 9 can be conducted by making the metal fine-particles 9 contact with the binding species 7 that have been immobilized on the metal fine-particles 3. The method of contacting the metal fine-particles 9 with the binding species 7 is not limited. For example, it is possible to immerse the semi-finished nano-composite with the binding species 7 bonded to the metal fine-particles 3, together with the matrix resin 1, in a treating liquid containing the metal fine-particles 9. It is also possible to sprinkle the treating liquid containing the metal fine-particles 9 on the semi-finished nano-composite by spraying, etc., or to coat the treating liquid with a suitable tool. Herein, for example, a metal colloidal solution can be well used as the treating liquid. In view of raising the efficiency of the bonding between the binding species 7 and the metal fine-particles 9, the immersion method is preferred among the above methods. In the immersion method, for example, a metal colloidal solution containing the metal fine-particles 9 is prepared, and a nano-composite 10 with metal fine-particles 9 bonded to the binding species 7 can be obtained by immersing, in the metal colloidal solution, the semi-finished nano-composite having the metal fine-particles 3 and the binding species 7. Moreover, when the immersion method is adopted, it is preferred that, for example, the treatment temperature is from 0°C to 50°C and the immersion time is from 1 min to 24 hours. After the metal fine-particles 9 are immobilized, it is feasible to conduct a cleaning step using pure water, etc., if required.

With the above steps, a nano-composite 10 having the constitution as shown in FIG. 1 can be fabricated. Moreover, even when the matrix resin 1 uses a resin other than the polyimide resin (polyamic acid resin), such a nano-composite 10 can also be fabricated based on the above fabrication method.

In the fabrication of the nano-composite 10, in addition to the above steps (1) to (4), arbitrary step can be conducted. For example, a binding species 11 can be further added to the metal fine-particles 9 of the nano-composite 10. In this case, the immobilization of the binding species 11 on the metal fine-particles 9 can be performed according to the step (4) of immobilizing the binding species. When a treating liquid obtained by dissolving the binding species 11 in a solvent is used to treat the surfaces of the metal fine-particles 9, the temperature is not particularly limited, and can be set in the range of 0-50°C, for example.

[Examples]

Next, this invention will be described specifically with examples, which are not intended to put any limitation on this invention. Moreover, any of the various measurements and evaluations is based on the corresponding one described below, as long as it is not specified in the examples of this invention.

[Measurement of the Mean Particle Diameter of Metal Fine-Particles]

In the measurement of the mean particle diameter of metal fine-particles, a microtome was (made by Leica Corporation, Ultra-Cut UTC Ultra-Microtome) used to make a ultra-thin slice from a cross section of a sample, and a transmission electron microscope (TEM; JEM-2000EX made by JEOL Ltd.) was used to observe the slice. Moreover, because a sample made on a glass substrate is difficult to observe with the above method, another sample made on the polyimide film under the same condition is used for the observation. Moreover, the mean particle diameter of the metal fine-particles is set to be an area mean diameter.

[Measurement of Diameter of Exposed Surfaces of Metal Fine-Particles]

The measurement of the diameter of the exposed surfaces of the metal fine-particles was conducted by observing the surface of the sample using a field-emission scanning electron microscope (FE-SEM from Hitachi High-Technologies Corporation).

[Measurement of the Absorption Spectrum of the Sample]

The absorption spectra of the fabricated samples were observed with UV-visible-near IR spectroscopy, using U-4000 manufactured by Hitachi, Ltd.

[Measurement of Light Transparency]

The light transparency was measured with UV-visible spectro-analysis, using UV-vis V-550 manufactured by JASCO Corporation.

(Method for Evaluating Variations of the Peak Wavelength and Peak Intensity)

The absorption spectra of the fabricated samples respectively in the environments of the atmosphere, water and ethanol are observed by UV-visible-near IR spectroscopy (using U-4000 manufactured by Hitachi, Ltd.). The variation of the peak wavelength is derived as follows. A graph was plotted, with the refractive indexes of the atmosphere, water and ethanol (the refractive index of the atmosphere was taken as 1, that of water taken as 1.33, and that of ethanol taken as 1.36) as the transverse axis, and with the wavelengths at the peak tops of the peak waveforms of the samples observed in the atmosphere, water and ethanol, respectively, as the vertical axis. The variation of the peak wavelength per unit variation of the refractive index was then derived as the slope of the straight line obtained with a least squares method. The variation of the peak intensity is derived as follows. A graph was plotted, with the refractive indexes of the atmosphere, water and ethanol (the refractive index of the atmosphere was taken as 1, that of water taken as 1.33, and that of ethanol taken as 1.36) as the transverse axis, and with the intensities at the peak tops of the peak waveforms of the samples observed in the atmosphere, water and ethanol, respectively, as the vertical axis. The variation of the peak intensity per unit variation of the refractive index was then derived as the slope of the straight line obtained with a least squares method. For obtaining a good LSPR effect, the variation of the peak intensity per unit variation of the refractive index was evaluated as "good" when it was greater than or equal to 0.22 nm of Fabrication Example 2 which was taken as a standard, and was evaluated as "excellent" when it was equal to 0.42 nm.

[Measurement of Water Absorption Ratio]

In the measurement of the water absorption ration, the sample was dried at 80°C for 2 hours, and the mass a of the dried sample was measured. Then, the dried sample was placed in an environment of 23°C and 50% RH for 24 hours (environment test), and then the mass b of the sample was measured. From such measured masses of the sample, the water absorption ratio was calculated by the following equation (A).

$$[\text{Water absorption ratio (\%)}]=\{(\text{weight b}-\text{weight a})/\text{weight a}\}\times 100 \qquad (A)$$

Synthesis Example 1

In a separable flask of 500 ml, 15.24 g (47.6 mmol) of 2,2'-bis(trifluoromethyl)-4,4'-diaminobiphenyl (TFMB)

was dissolved in 170g of DMAc. Next, the solution was added with 14.76 g (47.6 mmol) of 4,4'-oxydiphthalic anhydride (ODPA) under a nitrogen flow, and was further stirred at room temperature for 4 hours to conduct a polymerization reaction, so that a colorless thick polyamic acid resin solution $S_1$ was obtained. The viscosity of the obtained polyamic acid solution $S_1$ was measured using an E-type viscometer (DV-II+Pro CP type, made by Brookfield Corporation) to be 3251 cP (25°C). The weight-average molecular weight (Mw) was measured with gel permeation chromatography (GPC, using HLC-8220GPC made by Tosoh Corporation) to be 163,900.

The obtained polyamic acid resin solution $S_1$ was coated on a stainless substrate, dried at 125°C for 3 min, and then thermally treated stepwise at 160°C for 2 min, at 190°C for 30 min, at 200°C for 30 min, at 220°C for 3 min and then at 280°C, 320°C and 360°C respectively for 1 min to finish the imidization, so that a polyimide film laminated on a stainless substrate is obtained. The polyimide film was peeled from the stainless substrate to obtain a polyimide film $P_1$ of 10 $\mu$m thick. The transmittance of this film under 400 nm was 95%, and the mean transmittance of visible light was 96%.

Fabrication Example 1

A test plate of 10 cm×10 cm (0.7 mm thick) made of an alkali-free glass (AN-100 produced by Asahi Glass Co., Ltd.), was treated with a 5 N aqueous solution of sodium hydroxide of 50°C for 5 min. Next, the glass substrate of the test plate is cleaned by pure water, dried, and then immersed in a 1 wt% aqueous solution of 3-aminopropyltrimethoxysilane (abbreviated to "$\gamma$-APS" hereafter). The glass substrate was dried after being taken out from the aqueous $\gamma$-APS solution, and then heated at 150°C for 5 min to fabricate a glass substrate G1.

Fabrication Example 2

A test plate of 10 cm×10 cm (0.7 mm thick) made of an alkali-free glass (AN-100 produced by Asahi Glass Co., Ltd.) was surface-treated with vacuum evaporation at a pressure of $1\times10^{-5}$ Pa or lower to form a metal gold film of about 5 nm thick on the glass substrate. The substrate is thermally treated at 500°C for 1 hour to convert the metal gold film on the glass substrate into metal gold fine-particles, so that a test plate with metal gold fine-particles adhering to a glass substrate in a dispersion state was made. The characteristics of the metal gold fine-particles on the test plate are as follows.

The shape was a semi-spherical shape, the mean particle diameter was about 32 nm, the minimal particle diameter was about 9.5 nm, the maximal particle diameter was about 61 nm, the mean value of the inter-particle distances was about 13 nm, and the area fraction of the total gold fine-particles relative to the surface area of the test plate was about 19%. Moreover, the absorption spectrum of the LSPR of the test plate was observed to have an absorption peak with a peak top at 559 nm and a half-height width of 75 nm. The peak wavelength variation and the peak intensity variation per unit variation of the refractive index of the observed absorption peak were 52 nm and 0.22, respectively.

Fabrication Example 3

3 mg of a powder reagent of Biotin N-succinimide (Biotin Sulfo-OSu, produced by Dojindo Molecular Technologies, Inc.) was dissolved in 3 ml of a phosphoric acid-buffered saline as a mixed aqueous solution of 150 mM of sodium chloride, 7.5 mM of disodium hydrogenphosphate, and 2.9 mM of sodium dihydrogenphosphate, so that a Biotin solution 3 of 1 mg/ml was prepared.

Fabrication Example 4

1 mg of a powder reagent of Avidin (Avidin from egg white, produced by Nacalai Tesque, Inc.) was dissolved in 10 ml of a phosphoric acid-buffered saline as a mixed aqueous solution of 150 mM of sodium chloride, 7.5 mM of disodium hydrogenphosphate, and 2.9 mM of sodium dihydrogenphosphate, so that an Avidin solution 4 of 1.47 $\mu$M was prepared.

[Example 1]

<Fabrication of A Nano-Composite Film in which the First Metal Fine-Particles were dispersed>

0.174 g of chloroauric acid tetrahydrate dissolved in 17.33 g of DMAc was added to 2.67 g of the polyamic acid resin solution $S_1$ obtained in Synthesis Example, and the mixture was stirred in a nitrogen atmosphere for 15 min at room temperature to prepare a polyamic acid resin solution containing a gold complex. The obtained polyamic acid resin solution containing a gold complex was coated on the glass substrate G1 of Fabrication Example 1 using a spin coater

(Spincoater 1H-DX2, made by Mikasa Co., Ltd.), and was then dried at 70°C for 3 min and at 130°C for 20 min to form on the glass substrate G1 a polyamic acid resin film of 50 nm thick that contained a gold complex.
The polyamic acid resin film containing a gold complex was thermally treated at 300°C for 10 min, so that a nano-composite film 1a (30 nm thick) in which metal gold fine-particles showing a red color were dispersed was fabricated. The first metal gold fine-particles formed in the nano-composite film 1a were dispersed entirely independent from each other in the regions from the surface layer portion of the film along the thickness direction, with a distance greater than or equal to the particle diameter of the larger one of neighboring metal gold fine-particles. Moreover, the characteristics of the first metal gold fine-particles formed in the film are as follows.
The shape was a substantially spherical shape, the mean particle diameter was about 4.2 nm, the minimal particle diameter was about 3.0 nm, the maximal particle diameter was about 9.8 nm, the volume fraction relative to the nano-composite film 1a was about 1.35%, and the mean value of the inter-particle distances was about 17.4 nm.
Moreover, the absorption spectrum of the LSPR of the first metal gold fine-particles of the nano-composite film 1a was observed to have an absorption peak with a peak top at 544 nm and a half-height width of 78 nm.

<Step of Etching the Nano-Composite>

A vacuum plasma apparatus (Plasma Cleaner VE-1500II, manufactures by Mori Engineering Co., Ltd.) was used to remove a region of 7 nm thick of the nano-composite film 1a from the surface side thereof, so that a nano-composite film 1b was obtained. A part of the first metal gold fine-particles were exposed at the surface of the film at the surface side, and were identified to have a mean value of 3.8 nm for the diameters of the exposed surfaces of the metal gold fine-particles. At this moment, the area fraction of the total exposed portions of the first metal gold fine-particles relative to the surface area of the nano-composite film 1b was 1.08%. Moreover, the absorption spectrum of the LSPR of the first metal gold fine-particles of the nano-composite film 1b was observed to have an absorption peak with a peak top at 525 nm and a half-height width of 68 nm. The peak wavelength variation and the peak intensity variation per unit variation of the refractive index of the observed absorption peak were 10.3 nm and 0.02, respectively. Moreover, the surface observation image of the nano-composite 1b is shown in FIG. 5.

<Step of Immobilizing Binding Species>

Next, the nano-composite film 1b was immersed in a 0.1 mM (0.1 mmol/L) ethanol solution of the hydrochloric salt of aminoundecanethiol as a binding species and treated at -6°C for 2 hours, and was then cleaned using ethanol. Then, the nano-composite 1b was immersed in a 100 mM aqueous solution of potassium hydroxide and treated at 23°C for 30 sec, and was then cleaned by pure water and dried. Thereby, the ammonium group of the hydrochloric salt of aminoundecanethiol was converted to an amino group, and a nano-composite film 1c was prepared.

<Step of Immobilizing Second Metal Fine-Particles via Binding Species>

The nano-composite film 1c obtained as above was immersed in a metal gold colloidal solution 1 (produced by Tanaka Kikinzoku Inc., with a metal gold content of 0.007 wt%, a mean particle diameter of the metal gold colloidal particles of about 80 nm, a maximal particle diameter of 141.8 nm, and a minimal particle diameter of 50.8 nm) and treated under stirring at 23°C for 2 hours, and was then cleaned by pure water and dried. Thereby, second metal gold fine-particles formed from the metal gold colloidal particles are immobilized on the first metal gold fine-particles of the nano-composite film 1c, so that a nano-composite film 1d was obtained. As shown in FIG. 6, the second metal gold fine-particles of the nano-composite film 1d do not overlap with each other, and are in a state of being dispersed substantially uniformly in a plane. Dispersion unevenness was not identified. Moreover, the absorption spectrum of the LSPR of the second metal gold fine-particles of the nano-composite film 1d was observed to have an absorption peak with a peak top at 514 nm and a half-height width of 57 nm. The peak wavelength variation and the peak intensity variation per unit variation of the refractive index of the observed absorption peak were 63.8 nm and 0.42, respectively.

[Example 2]

A nano-composite film 2d was obtained as in Example 1 except that the metal gold colloidal solution 1 used in Example 1 was replaced by a metal gold colloidal solution 2 (produced by Tanaka Kikinzoku Inc., with a metal gold content of 0.007 wt%, a mean particle diameter of the metal gold colloidal particles of about 50 nm, a maximal particle diameter of 91.3 nm, and a minimal particle diameter of 32.6 nm). The second metal gold fine-particles of the nano-composite film 2d do not overlap with each other, and are in a state of being dispersed substantially uniformly in a plane. Dispersion unevenness was not identified. Moreover, the absorption spectrum of the LSPR of the second metal gold fine-particles of the nano-composite film 2d was observed to have an absorption peak with a peak top at 514 nm and a

half-height width of 57 nm. The peak wavelength variation and the peak intensity variation per unit variation of the refractive index of the observed absorption peak were 49.4 nm and 0.33, respectively.

[Example 3]

A nano-composite film 3d was obtained as in Example 1 except that the step of using the metal gold colloidal solution 1 to treat under stirring at 23°C for 2 hours in Example 1 was replaced by a step of using a metal gold colloidal solution 3 (produced by Tanaka Kikinzoku Inc., with a metal gold content of 0.007 wt%, a mean particle diameter of the metal gold colloidal particles of about 100 nm, a maximal particle diameter of 164.2 nm, and a minimal particle diameter of 68.1 nm) to treat under stirring at 23°C for 24 hours. The second metal gold fine-particles of the nano-composite film 3d do not overlap with each other, and are in a state of being dispersed substantially uniformly in a plane. Dispersion unevenness was not identified. Moreover, the absorption spectrum of the LSPR of the second metal gold fine-particles of the nano-composite film 3d was observed to have an absorption peak with a peak top at 513 nm and a half-height width of 58 nm. The peak wavelength variation and the peak intensity variation per unit variation of the refractive index of the observed absorption peak were 52.3 nm and 0.61, respectively.

[Example 4]

A nano-composite film 4d was obtained as in Example 1 except that the step of using the metal gold colloidal solution 1 to treat under stirring at 23°C for 2 hours in Example 1 was replaced by a step of using a metal gold colloidal solution 4 (produced by Tanaka Kikinzoku Inc., with a metal gold content of 0.007 wt%, a mean particle diameter of the metal gold colloidal particles of about 150 nm, a maximal particle diameter of 220.2 nm, and a minimal particle diameter of 105.7 nm) to treat under stirring at 23°C for 24 hours. The second metal gold fine-particles of the nano-composite film 4d do not overlap with each other, and are in a state of being dispersed substantially uniformly in a plane. Dispersion unevenness was not identified. Moreover, the absorption spectrum of the LSPR of the second metal gold fine-particles of the nano-composite film 4d was observed to have an absorption peak with a peak top at 515 nm and a half-height width of 58 nm. The peak wavelength variation and the peak intensity variation per unit variation of the refractive index of the observed absorption peak were 69.5 nm and 0.75, respectively.

[Example 5]

<Fabrication of Nano-Composite Film in which First Metal Fine-particles are Dispersed>

A nano-composite film 5a of 30 nm thick, in which metal gold fine-particles were dispersed, was fabricated as in Example 1 except that the use of 0.174 g of chloroauric acid tetrahydrate dissolved in 17.33 g of DMAc was replaced by the use of 0.087 g of chloroauric acid tetrahydrate dissolved in 17.33 g of DMAc. The first metal gold fine-particles formed in the nano-composite film 5a were dispersed entirely independently from each other in the regions from the surface layer portion of the film along the thickness direction, with a distance greater than or equal to the particle diameter of the larger one of neighboring metal gold fine-particles. Moreover, the characteristics of the first metal gold fine-particles formed in the film are as follows.
The shape was a substantially spherical shape, the mean particle diameter was about 3.8 nm, the minimal particle diameter was about 2.8 nm, the maximal particle diameter was about 9.5 nm, the volume fraction relative to the nano-composite film 5a was about 0.682%, and the mean value of the inter-particle distances was about 12.4 nm.
Moreover, the absorption spectrum of the LSPR of the first metal gold fine-particles of the nano-composite film 5a was observed to have an absorption peak with a peak top at 544 nm and a half-height width of 76 nm.

<Step of etching the Nano-Composite>

A nano-composite film 5b was obtained by conducting etching in the same way of Example 1. On the surface at the surface side of the film, a part of the first metal gold fine-particles were exposed, and the averaged diameter of the exposed surfaces of the metal gold fine-particles was identified to be about 3.5 nm. At this moment, the area fraction of the total exposed portions of the first metal gold fine-particles relative to the surface area of the nano-composite film 5b was 0.50%. Moreover, the absorption spectrum of the LSPR of the first metal gold fine-particles of the nano-composite film 5b was observed to have an absorption peak with a peak top at 534 nm and a half-height width of 68 nm. The peak wavelength variation and the peak intensity variation per unit variation of the refractive index of the observed absorption peak were 7.5 nm and 0.001, respectively.

<Step of Immobilizing Binding Species>

A nano-composite film 5c was prepared by immobilizing the binding species in the same way of Example 1

<Step of Immobilizing Second Metal Fine-Particles via Binding Species>

In the same way of Example 1, the nano-composite film 5c was immersed in the metal gold colloidal solution 1 to obtain a nano-composite film 5d. The second metal gold fine-particles of the nano-composite film 5d do not overlap with each other, and are in a state of being dispersed substantially uniformly in a plane. Dispersion unevenness was not identified. Moreover, the absorption spectrum of the LSPR of the second metal gold fine-particles of the nano-composite film 5d was observed to have an absorption peak with a peak top at 512 nm and a half-height width of 57 nm. The peak wavelength variation and the peak intensity variation per unit variation of the refractive index of the observed absorption peak were 73.7 nm and 0.31, respectively.

[Example 6]

A nano-composite film 6d was obtained as in Example 5 except that the step of using the metal gold colloidal solution 1 to treat under stirring at 23°C for 2 hours in Example 5 was replaced by a step of using the metal gold colloidal solution 3 (produced by Tanaka Kikinzoku Inc., with a metal gold content of 0.007 wt% and a mean particle diameter of the metal gold colloidal particles of about 100 nm) to treat under stirring at 23°C for 24 hours. The second metal gold fine-particles of the nano-composite film 6d do not overlap with each other, and are in a state of being dispersed substantially uniformly in a plane. Dispersion unevenness was not identified. Moreover, the absorption spectrum of the LSPR of the second metal gold fine-particles of the nano-composite film 6d was observed to have an absorption peak with a peak top at 514 nm and a half-height width of 57 nm. The peak wavelength variation and the peak intensity variation per unit variation of the refractive index of the observed absorption peak were 66.7 nm and 0.48, respectively.

[Example 7]

A nano-composite film 7d was obtained as in Example 5 except that the step of using the metal gold colloidal solution 1 to treat under stirring at 23°C for 2 hours in Example 5 was replaced by a step of using the metal gold colloidal solution 4 (produced by Tanaka Kikinzoku Inc., with a metal gold content of 0.007 wt% and a mean particle diameter of the metal gold colloidal particles of about 150 nm) to treat under stirring at 23°C for 24 hours. The second metal gold fine-particles of the nano-composite film 7d do not overlap with each other, and are in a state of being dispersed substantially uniformly in a plane. Dispersion unevenness was not identified. Moreover, the absorption spectrum of the LSPR of the second metal gold fine-particles of the nano-composite film 7d was observed to have an absorption peak with a peak top at 522 nm and a half-height width of 60 nm. The peak wavelength variation and the peak intensity variation per unit variation of the refractive index of the observed absorption peak were 55.5 nm and 0.38, respectively.

[Example 8]

<Fabrication of Nano-Composite Film in which First Metal Fine-particles are Dispersed>

A nano-composite film 8a of 30 nm thick, in which metal gold fine-particles were dispersed, was fabricated as in Example 1 except that the use of 0.174 g of chloroauric acid tetrahydrate dissolved in 17.33 g of DMAc was replaced by the use of 0.058 g of chloroauric acid tetrahydrate dissolved in 17.33 g of DMAc. The first metal gold fine-particles formed in the nano-composite film 8a were dispersed entirely independently from each other in the regions from the surface layer portion of the film along the thickness direction, with a distance greater than or equal to the particle diameter of the larger one of neighboring metal gold fine-particles. Moreover, the characteristics of the first metal gold fine-particles formed in the film are as follows.
The shape was a substantially spherical shape, the mean particle diameter was about 3.7 nm, the minimal particle diameter was about 2.8 nm, the maximal particle diameter was about 9.1 nm, the volume fraction relative to the nano-composite film 8a was about 0.456%, and the mean value of the inter-particle distances was about 14.3 nm.
Moreover, the absorption spectrum of the LSPR of the first metal gold fine-particles of the nano-composite film 8a was observed to have an absorption peak with a peak top at 543 nm and a half-height width of 79 nm.

<Step of etching the Nano-Composite>

A nano-composite film 8b was obtained by conducting etching in the same way of Example 1. On the surface

at the surface side of the film, a part of the first metal gold fine-particles were exposed, and the averaged diameter of the exposed surfaces of the metal gold fine-particles was identified to be about 3.5 nm. At this moment, the area fraction of the total exposed portions of the first metal gold fine-particles relative to the surface area of the nano-composite film 8b was 0.42%. Moreover, from the absorption spectrum of the LSPR of the first metal gold fine-particles of the nano-composite film 8b, a peak top and a half-height width were difficult to measure.

<Step of Immobilizing Binding Species>

A nano-composite film 8c was prepared by immobilizing the binding species in the same way of Example 1

<Step of Immobilizing Second Metal Gold Fine-Particles via Binding Species>

In the same way of Example 1, the nano-composite film 8c was immersed in the metal gold colloidal solution 1 to obtain a nano-composite film 8d. The second metal gold fine-particles of the nano-composite film 8d do not overlap with each other, and are in a state of being dispersed substantially uniformly in a plane. Dispersion unevenness was not identified. Moreover, the absorption spectrum of the LSPR of the second metal gold fine-particles of the nano-composite film 8d was observed to have an absorption peak with a peak top at 514 nm and a half-height width of 58 nm. The peak wavelength variation and the peak intensity variation per unit variation of the refractive index of the observed absorption peak were 65.4 nm and 0.35, respectively.

[Example 9]

A nano-composite film 9d was obtained as in Example 8 except that the use of the metal gold colloidal solution 1 in Example 8 was replaced by the use of the metal gold colloidal solution 2 (produced by Tanaka Kikinzoku Inc., with a metal gold content of 0.007 wt% and a mean particle diameter of the metal gold colloidal particles of about 50 nm). The second metal gold fine-particles of the nano-composite film 9d do not overlap with each other, and are in a state of being dispersed substantially uniformly in a plane. Dispersion unevenness was not identified. Moreover, the absorption spectrum of the LSPR of the second metal gold fine-particles of the nano-composite film 9d was observed to have an absorption peak with a peak top at 514 nm and a half-height width of 57 nm. The peak wavelength variation and the peak intensity variation per unit variation of the refractive index of the observed absorption peak were 56.4 nm and 0.15, respectively.

[Example 10]

A nano-composite film 10d was obtained as in Example 8 except that the step of using the metal gold colloidal solution 1 to treat under stirring at 23°C for 2 hours in Example 8 was replaced by a step of using the metal gold colloidal solution 3 (produced by Tanaka Kikinzoku Inc., with a metal gold content of 0.007 wt% and a mean particle diameter of the metal gold colloidal particles of about 100 nm) to treat under stirring at 23°C for 24 hours. The second metal gold fine-particles of the nano-composite film 10d do not overlap with each other, and are in a state of being dispersed substantially uniformly in a plane. Dispersion unevenness was either not identified. Moreover, the absorption spectrum of the LSPR of the second metal gold fine-particles of the nano-composite film 10d was observed to have an absorption peak with a peak top at 514 nm and a half-height width of 59 nm. The peak wavelength variation and the peak intensity variation per unit variation of the refractive index of the observed absorption peak were 66.7 nm and 0.49, respectively.

[Example 11]

<Fabrication of Nano-Composite Film in which First Metal Fine-particles are Dispersed>

A nano-composite film 11a of 30 nm thick, in which metal gold fine-particles were dispersed, was fabricated as in Example 1 except that the use of 0.174 g of chloroauric acid tetrahydrate dissolved in 17.33 g of DMAc was replaced by the use of 0.522 g of chloroauric acid tetrahydrate dissolved in 17.33 g of DMAc. The first metal gold fine-particles formed in the nano-composite film 11a were dispersed entirely independently from each other in the regions from the surface layer portion of the film along the thickness direction, with a distance greater than or equal to the particle diameter of the larger one of neighboring metal gold fine-particles, and with a side-by-side arrangement in a single layer. Moreover, the characteristics of the first metal gold fine-particles formed in the film are as follows.
The shape was a substantially spherical shape, the mean particle diameter was about 20 nm, the minimal particle diameter was about 12 nm, the maximal particle diameter was about 26 nm, the volume fraction relative to the nano-composite film 11a was about 3.96%, and the mean value of the inter-particle distances was about 25 nm.

Moreover, the absorption spectrum of the LSPR of the first metal gold fine-particles of the nano-composite film 11a was observed to have an absorption peak with a peak top at 546 nm and a half-height width of 102 nm.

<Step of etching the Nano-Composite>

A nano-composite film 11b was obtained by conducting etching in the same way of Example 1. On the surface at the surface side of the film, a part of the first metal gold fine-particles were exposed, and the averaged diameter of the exposed surfaces of the metal gold fine-particles was identified to be about 17.9 nm. At this moment, the area fraction of the total exposed portions of the first metal gold fine-particles relative to the surface area of the nano-composite film 11b was 3.2%. Moreover, the absorption spectrum of the LSPR of the second metal gold fine-particles of the nano-composite film 11b was observed to have an absorption peak with a peak top at 528 nm and a half-height width of 80 nm. The peak wavelength variation and the peak intensity variation per unit variation of the refractive index of the observed absorption peak were -5.8 nm and 0.02, respectively.

<Step of Immobilizing Binding Species>

A nano-composite film 11c was prepared by immobilizing the binding species in the same way of Example 1

<Step of Immobilizing Second Metal Gold Fine-Particles via Binding Species>

In the same way of Example 1, the nano-composite film 11c was immersed in the metal gold colloidal solution 1 to obtain a nano-composite film 11d. The second metal gold fine-particles of the nano-composite film 11d do not overlap with each other, and are in a state of being dispersed substantially uniformly in a plane. Dispersion unevenness was not identified. Moreover, the absorption spectrum of the LSPR of the second metal gold fine-particles of the nano-composite film 11d was observed to have an absorption peak with a peak top at 524 nm and a half-height width of 72 nm. The peak wavelength variation and the peak intensity variation per unit variation of the refractive index of the observed absorption peak were 56.4 nm and 0.24, respectively.

[Example 12]

A nano-composite film 12d was obtained as in Example 11 except that the step of using the metal gold colloidal solution 1 to treat under stirring at 23°C for 2 hours in Example 11 was replaced by a step of using the metal gold colloidal solution 3 (produced by Tanaka Kikinzoku Inc., with a metal gold content of 0.007 wt% and a mean particle diameter of the metal gold colloidal particles of about 100 nm) to treat under stirring at 23°C for 24 hours. The second metal gold fine-particles of the nano-composite film 12d do not overlap with each other, and are in a state of being dispersed substantially uniformly in a plane. Dispersion unevenness was not identified. Moreover, the absorption spectrum of the LSPR of the second metal gold fine-particles of the nano-composite film 12d was observed to have an absorption peak with a peak top at 514 nm and a half-height width of 58 nm. The peak wavelength variation and the peak intensity variation per unit variation of the refractive index of the observed absorption peak were 56.4 nm and 0.63, respectively.

[Example 13]

A nano-composite film 13d was obtained as in Example 11 except that the step of using the metal gold colloidal solution 1 to treat under stirring at 23°C for 2 hours in Example 11 was replaced by a step of using the metal gold colloidal solution 4 (produced by Tanaka Kikinzoku Inc., with a metal gold content of 0.007 wt% and a mean particle diameter of the metal gold colloidal particles of about 150 nm) to treat under stirring at 23°C for 24 hours. The second metal gold fine-particles of the nano-composite film 13d do not overlap with each other, and are in a state of being dispersed substantially uniformly in a plane. Dispersion unevenness was not identified. Moreover, the absorption spectrum of the LSPR of the second metal gold fine-particles of the nano-composite film 13d was observed to have an absorption peak with a peak top at 526 nm and a half-height width of 60 nm. The peak wavelength variation and the peak intensity variation per unit variation of the refractive index of the observed absorption peak were 51.9 nm and 0.25, respectively.

[Example 14]

A nano-composite film 14d with immobilized second metal gold fine-particles was obtained in the same way of Example 1.

Next, the nano-composite film 14d was immersed in a 0.1 mM (0.1 mmol/L) ethanol solution of the hydrochloric salt of aminoundecanethiol as a binding species and treat at 23°C for 2 hours, and was then cleaned with ethanol and

dried to prepare a nano-composite film 14e.

Next, the nano-composite film 14e was immersed in the Biotin solution 3 of Fabrication Example 3 and treat at 23°C for 2 hours, and was then cleaned with a phosphoric acid-buffered saline and then immersed in the phosphoric acid-buffered saline. Thereby, a nano-composite film 14e' was prepared with Biotin N-succinimidyl further immobilized on the binding species of the nano-composite film 14e. The absorption spectrum of the nano-composite film 14e' in the phosphoric acid-buffered saline was observed to have an absorption peak with a peak top at 535 nm, a half-height width of 58 nm, and a peak top absorbance of 0.250.

The nano-composite film 14e' was immersed in the Avidin solution 4 of Fabrication Example 4 and treat under stirring at 23°C for 2 hours, and was then cleaned with a phosphoric acid-buffered saline and then immersed in the phosphoric acid-buffered saline. Thereby, a nano-composite film 14e" was prepared with Avidin adsorbed by the Biotin moiety of the binding species of the nano-composite film 14e'. The absorption spectrum of the nano-composite film 14e" in the phosphoric acid-buffered saline was observed to have an absorption peak with a peak top at 539 nm, a half-height width of 58 nm, and a peak top absorbance of 0.278.

Reference Example 1

17.33 g of DMAc was added in 2.67 g of the polyamic acid resin solution $S_1$ obtained in Synthesis Example 1 to dilute the polyamic acid resin solution $S_1$. The resulting polyamic acid resin solution was coated on a glass substrate G1 of Fabrication Example 1 using a spin coater (Spincoater 1H-DX2, made by Mikasa Co., Ltd.), and was then dried at 70°C for 3 min and 130°C for 20 min, so that a polyamic acid resin film of 50 nm thick was formed on the glass substrate G1. By thermally treating the polyamic acid resin film at 300°C for 10 min, a colorless transparent polyimide film of 30 nm thick was fabricated.

<Step of Immobilizing Binding Species>

Next, the polyimide film was immersed in a 0.1 mM (0.1 mmol/L) ethanol solution of the hydrochloric salt of aminoundecanethiol as a binding species and treated at 23°C for 2 hours, and was then cleaned with ethanol and dried to prepare a polyimide film with an immobilized binding species.

<Step of Immobilizing Metal Colloidal Particles via Binding Species>

The polyimide film obtained as above was immersed in the metal gold colloidal solution 1 (produced by Tanaka Kikinzoku Inc., with a metal gold content of 0.007 wt% and a mean particle diameter of the metal gold colloidal particles of about 80 nm) and treated under stirring at 23°C for 2 hours, and was then cleaned by pure water and dried. The resulting polyimide film was found to have metal gold colloidal particles adhering to its surface in an aggregation state, as shown in FIG. 7. The absorption spectrum thereof was observed to have a broad absorption peak in the wavelength region of 600 nm to 800 nm, which was caused by the aggregated metal gold colloidal particles.

Reference Example 2

In the same way of Example 1, a nano-composite film in which metal gold fine-particles were dispersed was obtained, and a nano-composite film having been subjected to an etching step was fabricated.

<Step of Immobilizing Binding Species>

The nano-composite film obtained as above was immersed in a 0.1 mM (0.1 mmol/L) ethanol solution of the hydrochloric salt of aminoundecanethiol as a binding species and treated at 23°C for 2 hours, and was then cleaned with ethanol and dried. Next, the nano-composite film was immersed in a 100 mM aqueous solution of potassium hydroxide and treated at 23°C for 30 min, and was then cleaned with pure water and dried. Thereby, the ammonium group in the hydrochloric salt of aminoundecanethiol was converted to an amino group, and the binding species is immobilized all over the entire surface of the nano-composite film.

<Step of Immobilizing Metal Colloidal Particles via Binding Species>

The nano-composite film obtained as above was immersed in the metal gold colloidal solution 1 (produced by Tanaka Kikinzoku Inc., with a metal gold content of 0.007 wt% and a mean particle diameter of the metal gold colloidal particles of about 80 nm) and treated under stirring at 23°C for 2 hours, and was then cleaned by pure water and dried. The resulting nano-composite film was found to have metal gold colloidal particles adhering to its surface in an aggregation

state, as shown in FIG. 8. The absorption spectrum thereof was observed to have a broad absorption peak in the wavelength region of 600 nm to 800 nm that was caused by the aggregated metal gold colloidal particles.

It is clear from the above results that the nano-composite films obtained in Examples 1-14 exhibited a sufficient intensity of the absorption spectrum of the LSPR of the second metal gold fine-particles formed from metal gold colloidal particles, and a sharp absorption peak with a small half-height width. Therefore, it was confirmed that the nano-composite films allow a high-sensitivity detection when being used in certain applications, such as bio-sensors, etc.

Though this invention has been described with the above embodiments, the invention is not limited as such, and various modifications are allowed. This international application claims the priority benefits of Japanese Patent Application No. 2010-123225 filed on May 28, 2010, of which the entire contents are cited.

[Description of Reference Characters]

1: matrix resin; 3: metal fine-particle; 5: metal fine-particle layer; 7: binding species; 9: metal fine-particle; 10: nano-composite; 11: binding species; S: surface.

## Claims

1. A metal fine-particle composite, comprising:

    a matrix resin, and metal fine-particles immobilized to the matrix resin, wherein

    a) the metal fine-particles include a plurality of first metal fine-particles immobilized in the matrix resin, and second metal fine-particles indirectly immobilized on the first metal fine-particles,
    b) the first metal fine-particles are present independently without contacting with each other, and
    c) each of at least a part of the first metal fine-particles has a portion embedded in the matrix resin and another portion exposed outside of the matrix resin, while the second metal fine-particles are immobilized via a binding species immobilized on the another exposed portion.

2. The metal fine-particle composite of claim 1, wherein the first metal fine-particles have particle diameters in a range of 1 nm to 50 nm and a mean particle diameter greater than or equal to 3 nm, and the second metal fine-particles have a mean particle diameter in a range of 40 nm to 200 nm.

3. The metal fine-particle composite of claim 1 or 2, wherein the first metal fine-particles are present with a distance that is greater than or equal to a particle diameter of a larger one of two neighboring fine-particles among the first metal fine-particles.

4. The metal fine-particle composite of any one of claims 1-3, wherein another binding species having a functional group interacting with a specific substance is immobilized on surfaces of the second metal fine-particles.

5. The metal fine-particle composite of any one of claims 1-4, wherein the second metal fine-particles are formed from a metal colloidal.

6. A method for producing the metal fine-particle composite of any one of claims 1-5, the method comprising:

    A) a step of contacting the first metal fine-particles, each of which has a portion embedded in the matrix resin and another portion exposed outside of the matrix resin, with a treating liquid containing the binding species at a temperature of 20°C or lower to selectively binding the binding species to the another exposed portion; and
    B) a step of immobilizing the second metal fine-particles via the immobilized binding species.

7. The method of claim 6, further comprising, before the step A,

    C) a step of forming a resin film containing a metal ion or a metal salt;
    D) a step of thermally reducing the metal ion or the metal salt in the resin film to separate out the plurality of first metal fine-particles in the matrix resin and
    E) a step of etching a surface of the matrix resin to partially expose a surface of each of at least the part of the first metal fine-particles.

**8.** The method of claim 6 or 7, further comprising, after the step B,

F) a step of immobilizing, on surfaces of the second metal fine-particles, another binding species having a functional group interacting with a specific substance.

**9.** The method of any one of claims 6-8, wherein the step B uses a metal colloidal solution containing the second metal fine-particles in a form of metal colloidal.

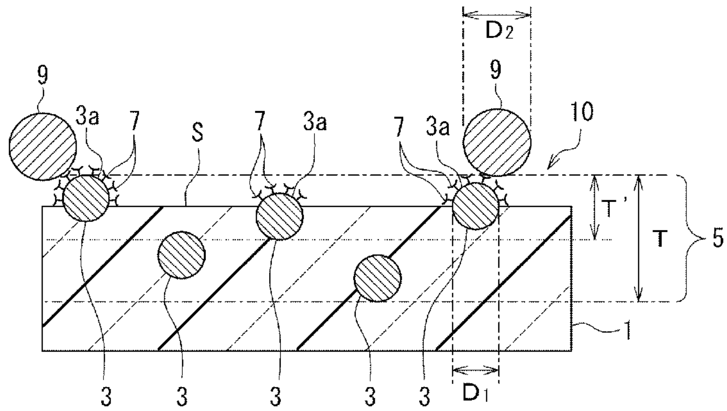


FIG. 1

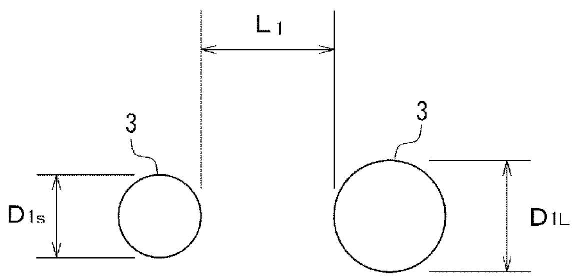


FIG. 2

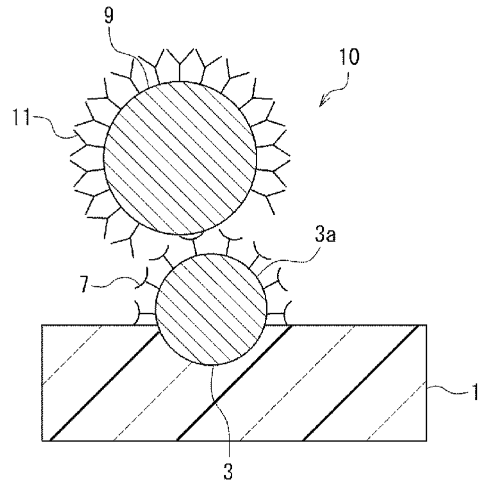


FIG. 3

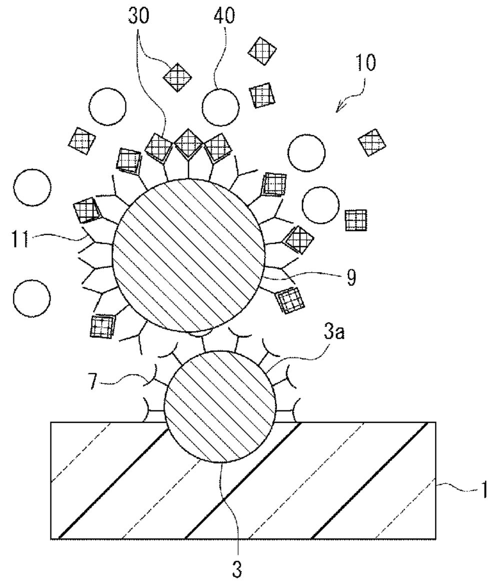


FIG. 4

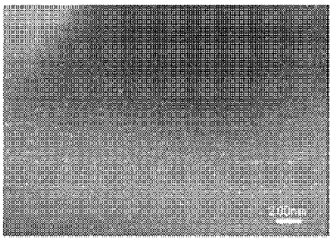

FIG. 5

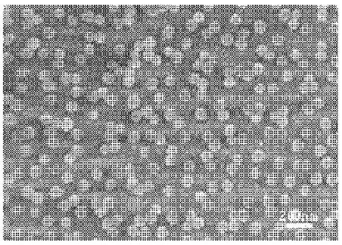

FIG. 6

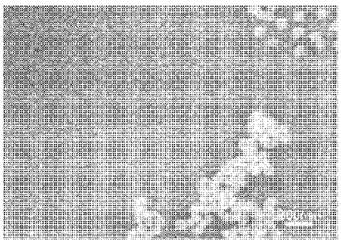

FIG. 7

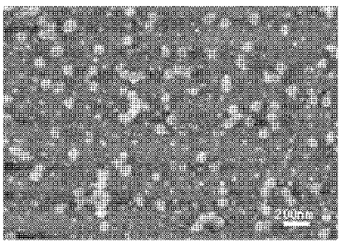

FIG. 8

## INTERNATIONAL SEARCH REPORT

International application No.
PCT/JP2011/061634

A. CLASSIFICATION OF SUBJECT MATTER
*G01N21/27*(2006.01)i, *B82Y15/00*(2011.01)i, *G01N33/543*(2006.01)i

According to International Patent Classification (IPC) or to both national classification and IPC

B. FIELDS SEARCHED

Minimum documentation searched (classification system followed by classification symbols)
G01N21/00-21/74, G01N33/543

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)
JSTPlus/JMEDPlus/JST7580(JDreamII)

C. DOCUMENTS CONSIDERED TO BE RELEVANT

| Category* | Citation of document, with indication, where appropriate, of the relevant passages | Relevant to claim No. |
|---|---|---|
| Y | JP 2008-157923 A (Canon Inc.), 10 July 2008 (10.07.2008), paragraphs [0066] to [0084], [0112] to [0136]; fig. 3 to 6 & US 2008/0130003 A1 | 1-9 |
| Y | WO 2007/122998 A1 (Japan Science and Technology Agency), 01 November 2007 (01.11.2007), paragraphs [0007], [0023] to [0041] & US 2009/0087582 A1 & EP 2003091 A2 & CN 101432224 A & KR 10-2009-0028503 A | 1-9 |

[X] Further documents are listed in the continuation of Box C. [ ] See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>11 August, 2011 (11.08.11) | Date of mailing of the international search report<br>23 August, 2011 (23.08.11) |
| Name and mailing address of the ISA/<br>Japanese Patent Office<br>Facsimile No. | Authorized officer<br><br>Telephone No. |

Form PCT/ISA/210 (second sheet) (July 2009)

**INTERNATIONAL SEARCH REPORT**

International application No.
PCT/JP2011/061634

C (Continuation). DOCUMENTS CONSIDERED TO BE RELEVANT

| Category* | Citation of document, with indication, where appropriate, of the relevant passages | Relevant to claim No. |
|---|---|---|
| Y | Pieper-Furst U, et.al., Gold nanoparticle-enhanced surface plasmon resonance measurement with a highly sensitive quantification for human tissue inhibitor of metalloproteinases-2, Analytica Chimica Acta, 2005.09.26, Volo.550, pp.69-76 | 5-9 |
| Y | JP 2006-030155 A (Yukio NAGASAKI), 02 February 2006 (02.02.2006), paragraphs [0035] to [0048] (Family: none) | 6-9 |
| A | Jeong H J, et.al., Gold Nanoparticle-Hybridaized "Nano-Sponge" Polymer Coatings to Enhance the Reliability and Sensitivity of Biosensors, Macromolecular Rapid Communications, 2009.07.01, Vol.30, No.13, pp.1109-1115 | 1-9 |
| A | JP 2007-508536 A (Commissariat A L'energie Atomique), 05 April 2007 (05.04.2007), entire text; all drawings & US 2007/0115474 A1 & EP 1670951 A & WO 2005/033335 A2 & FR 2860872 A | 1-9 |
| A | JP 2005-336538 A (Fuji Photo Film Co., Ltd.), 08 December 2005 (08.12.2005), entire text; all drawings & US 2005/0211566 A1 & EP 1580305 A2 | 1-9 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2006234472 A
- JP 2010123225 A


### Non-patent literature cited in the description


- **GRABAR, K. C. et al.** *Anal. Chem.,* 1995, vol. 67, 735-743
- **FREEMAN, R. G. et al.** *Science,* 1995, vol. 267, 1629-1632